# EUROPEAN PATENT APPLICATION

(11) **EP 2 657 267 A1**
(43) Date of publication of application: **30.10.2013**
(21) Application number: 10838729.1
(22) Date of filing: 22.12.2010
(51) Int. Cl.: C08G 18/04, C08G 71/00, G01N 33/50, C12N 15/63, A61K 47/34

(54) **BIOACTIVE SURFACE CAPABLE OF GENETICALLY MODIFYING BIOLOGICAL CELLS OR TISSUES AND USE THEREOF**

(71) Applicant: Fundacion Centro Nacional De Investigaciones Cardiovasculares Carlos III (CINC), 28029 Madrid (ES); Institut Quimic De Sarria Cets Fundacio Privada (IQS), 08017 Barcelona (ES); Universitat Ramon Llull, Fundacio Privada, 08022 Barcelona (ES)
(72) Inventor: GONZALEZ DE LA PEÑA, Manuel Angel, E-28029 Madrid (ES); RAMIREZ MARTINEZ, Juan Carlos, E-28029 Madrid (ES); BERNAD MIANA, Antonio, E-28029 Madrid (ES); BORROS GOMEZ, Salvador, E-08017 Barcelona (ES); HORNA TOMAS, David, E-08017 Barcelona (ES); CIFUENTES, Anna, E-08017 Barcelona (ES)
(74) Representative: ABG Patentes, S.L.
(86) International application number: PCT/ES2010/070863
(87) International publication number: WO 2011/076970

(57) **Abstract**

The invention relates to a bioactive surface comprising a polymer matrix, a connecting complex comprising at least one compound covalently bound to the surface of the polymer matrix, and a gene transfer vector bound to the connecting complex, where the compound of complex is covalently bound to the transfer vector using a group selected from among carboxyl, amino, isocyanate and hydroxyl, and to the use of the bioactive surface for transferring a nucleic acid to a cell. The invention also relates to an implantable device characterized in that at least part of the surface thereof is coated with said bioactive surface, and to the use of the implantable device for transferring a nucleic acid to a cell. In addition, the invention relates to a method for transferring a nucleic acid to a cell and to a kit for carrying out said method.

## Description

The present invention is comprised within the field of molecular biology and biomedicine. Specifically, the present invention relates to a bioactive surface comprising: a polymer matrix, a connecting complex comprising at least one compound covalently bound to the surface of the polymer matrix, and a gene transfer vector bound to the connecting complex, where the compound of the complex is covalently bound to the transfer vector by means of a group selected from a carboxyl group, an amino group, an isocyanate group and a hydroxyl group, and to the use of said bioactive surface for transferring a nucleic acid to a cell. The present invention also relates to an implantable device characterized in that at least one part of its surface is coated with said bioactive surface and to the use of said implantable device for transferring a nucleic acid to a cell. The present invention also relates to a method for transferring a nucleic acid to a cell and to a kit for carrying out said method.

### Prior State of the Art

Gene transfer has a great therapeutic potential both in gene therapy and in tissue engineering, in addition to being very useful in research. The limiting factor in the development of gene transfer-based applications is the lack of effective systems for introducing the genetic material in cells. For gene transfer to be effective, nucleic acid must be transported to the target cells without it degrading, being taken into said cells, escape the intracellular degradation systems, being transported to the nucleus for transcription, and finally being translated into a protein. The use of viral or non-viral vectors also facilitates overcoming many intracellular barriers in the gene transfer process, the use of biomaterials allows solving problems associated with the transport of genetic material to target cells.

Substrate-dependent gene transfer, also called reverse transfection, is based on immobilizing viral or non-viral vectors on a surface which allows cell adhesion. This strategy allows reducing genetic material degradation and increasing the effective vector concentration, therefore a lower amount of nucleic acid is necessary, with the subsequent reduction in cytotoxicity. On the other hand, a tool which allows spatially regulating gene transfer process is obtained by combining the immobilization of the vector with a surface pattern.

The immobilization of the genetic material to be transferred can be non-specific or specific. Some biomaterials allow gene transfer vectors to be adsorbed directly on the surface; in this case the vectors interact with said biomaterials by means of non-specific mechanisms such as hydrophobic interactions, electrostatic interactions and van der Waals forces. Alternatively, specific interactions can be introduced by means of functional groups in the vector and the polymer, such as avidin-biotin or antibody-antigen.

Collagen hydrogels and hyaluronic acid having neutravidin on their surface are capable of binding DNA complexes and biotinylated polyethylenimine cationic polymer complexes (Segura et. al. Biomaterials. 2005 May; 26(13): 1575-1584). The avidin-biotin system has also been used for immobilizing adenoviral vectors on chitosan supports (Hu et. al. J Gene Med. 2008 October; 10(10): 1102-1112). The immobilization of adenovirus on type I collagen gels modified with avidin using to that end an anti-adenovirus biotinylated antibody has also been described (Levy et. al. Gene Ther. 2001 May;8(9):659-67). Likewise, adenoviruses have been immobilized by means of specific antibodies bound directly to polyurethane films (Stachelek et. al. Gene Ther. 2004 Jan; 11(1):15-24). It is important to highlight that specific immobilization strategy-based implantable devices have been used for *in vivo* gene transfer (Levy et. al. Gene Ther. 2001 May; 8(9):659-67; Klugherz et. al. Hum Gene Ther. 2002 Feb 10;13(3):443-54; Abrahams et. al; Stroke. 2002 May;33(5):1376-82).

Regardless of the strategy used for immobilizing gene transfer vectors, vector immobilization and cellular uptake must be in equilibrium. The biomaterial used must be suitable for cell adhesion and also keep the gene transfer vector bound to the surface, but allowing cellular uptake of genetic material. If the bond is too weak the vectors will not be retained on the surface of the biomaterial for presentation to cells; in contrast, if vector immobilization is too strong, the uptake of genetic material may decrease significantly.

In conclusion, the enormous application potential of gene transfer in gene therapy and tissue engineering is significantly limited by the lack of effective, safe and controlled systems for gene transfer. The immobilization of genetic material in biomaterials is a strategy which allows improving efficacy and safety, as well as space-time control of the gene transfer process, provided that there is optimum equilibrium between the immobilization of genetic material and its cellular uptake.

### Description of the Invention

The present invention relates to a bioactive surface, providing an effective, safe and controlled system for gene transfer. This bioactive surface of the invention comprises:
a) a polymer matrix,
b) a connecting complex comprising at least one compound covalently bound to the surface of the polymer matrix (a), and
c) a gene transfer vector bound to the connecting complex, where the compound of complex (b) is covalently bound to the transfer vector by means of a group selected from a carboxyl group, an amino group, an isocyanate group and a hydroxyl group, and to the use of said bioactive surface for transferring a nucleic acid to a cell.

The present invention also relates to an implantable device characterized in that at least one part of its surface is coated with said bioactive surface and to the use of said implantable device for transferring a nucleic acid to a cell. Likewise, the present invention relates to a method for transferring a nucleic acid to a cell and to a kit for carrying out said method.

The inventors used polystyrene matrices the surface of which had been previously modified by means of the covalent bond with pentafluorophenyl methacrylate (PFM). Treating the surface of the matrices with PFM proved effective for binding ineffective particles containing amino groups, particularly lentiviral and adenoviral particles, to said surface. These dishes treated with PFM and said particles are capable of genetically transducing human cells in a more effective manner than the same particles adsorbed in a non-specific manner on dishes which had not been treated with said molecule.

Therefore, a first aspect of the invention relates to a bioactive surface (hereinafter, bioactive surface of the invention) comprising:
a) a polymer matrix,
b) a connecting complex comprising at least one compound covalently bound to the surface of the polymer matrix (a), and
c) a gene transfer vector bound to the connecting complex, where the compound of complex (b) is covalently bound to the transfer vector by means of a group selected from a carboxyl group, an amino group, an isocyanate group and a hydroxyl group.

As used herein, the term "bioactive surface" refers to any surface of a material with the capacity to transfer a biomolecule to a cell. Said biomolecule is preferably a nucleic acid. Therefore, in a preferred embodiment this term refers to any surface of a material having the capacity to genetically modify biological tissues or cells; the bioactive surface is therefore a gene transfer complex.

As used herein, "polymer matrix" is understood as any composite material that is biocompatible, biodegradable or non-biodegradable, formed by fibers, particles, or filaments embedded during the resinous or malleable phase. The polymer matrix of the bioactive surface of the invention is preferably selected from the list comprising: polystyrene, polycarbonate, poly(ethylene carbonate), polyethylene, polyolefins, poly(diol citrate), polyfumarate, polylactides, polycaprolactone, polyacrylamides, polysiloxanes, polyesters, polyamides, glycolic/lactic acid copolymers, polyurethane or any combination thereof. In a more preferred embodiment, the polymer matrix of the bioactive surface of the invention comprises polystyrene.

As used herein, the term "connecting complex" refers to a compound capable of binding the gene transfer vector to the polymer matrix by means of forming a covalent bond both with the polymer matrix and with the transfer vector. The connecting complex of the bioactive surface of the invention comprises at least one compound containing a group selected from a carboxyl group, an amino group, an isocyanate group and a hydroxyl group anchored to the surface of the polymer matrix.

The compound bound to the matrix preferably has the following formula (I): 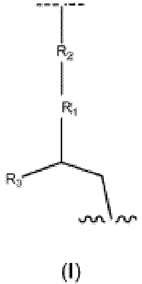 where:
R₁ is a radical which may or may not exist and if it exists, it is selected from a group comprising a (C₁-C₆) alkyl, a (C₁-C₆) alkenyl, a cycloalkyl or an aryl; preferably R₁ does not exist,
R₂ is a carboxyl group (-COO-), an amino group (-NH-), an ether group (-O-) or an isocyanate group (-CON-), preferably R₂ is a carboxyl group,
R₃ is a hydrogen, a (C₁-C₆) alkyl or a (C₁-C₆) alkene, preferably R₃ is a hydrogen or a methyl group,
----- represents the binding of the compound of formula (I) to the gene transfer vector, and
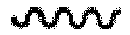 represents the binding of the compound of formula (I) to the polymer matrix.

In the present invention, the term "alkyl" refers to radicals of linear or branched hydrocarbon chains having from 1 to 10 carbon atoms, preferably from 1 to 4, and binding to the rest of the molecule by means of a single bond, for example, methyl, ethyl, *n*-propyl, *i*-propyl, *n*-butyl, *tert*-butyl, *sec-*butyl, *n*-pentyl, *n*-hexyl, etc. The alkyl groups can optionally be substituted with one or more substituents such as halogen, hydroxyl, alkoxyl, carboxyl, carbonyl, cyano, acyl, alkoxycarbonyl, amino, niter, mercapto and alkylthio.

The term "alkenyl" refers to radicals of hydrocarbon chains containing one or more carbon-carbon double bonds, for example, vinyl, 1-propenyl, allyl, isoprenyl, 2-butenyl, 1,3-butadienyl, etc. Alkenyl radicals can optionally be substituted with one or more substituents such as halo, hydroxyl, alkoxyl, carboxyl, cyano, carbonyl, acyl, alkoxycarbonyl, amino, niter, mercapto and alkylthio.

In the present invention, "cycloalkyl" refers to a stable 3 to 10-member monocyclic or bicyclic radical, which is saturated or partially saturated and which only consists of carbon and hydrogen atoms, such as cyclopentyl, cyclohexyl or adamantyl and which can optionally be substituted with one or more groups such as alkyl, halogen, hydroxyl, alkoxyl, carboxyl, cyano, carbonyl, acyl, alkoxycarbonyl, amino, niter, mercapto and alkylthio.

In the present invention, the term "aryl" refers to a phenyl, naphthyl, indenyl, phenanthryl and anthracyl radical. The aryl radical can optionally be substituted with one or more substituents such as alkyl, haloalkyl, aminoalkyl, dialkylamino, hydroxyl, alkoxyl, phenyl, mercapto, halogen, niter, cyano and alkoxycarbonyl.

The compound of formula (I) is preferably 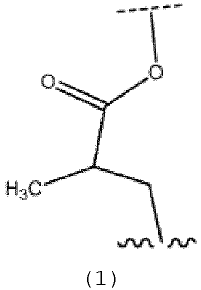 where: ----- and 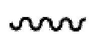 have been described previously. This compound (1) preferably originates from PFM (pentafluorophenyl methacrylate).

In a preferred embodiment, the compound covalently bound to the polymer matrix is bound to the transfer vector by means of a carboxyl group. In a preferred embodiment, the compound covalently bound to the matrix is bound to the transfer vector by means of an amino group. In a preferred embodiment, the compound covalently bound to the polymer matrix is bound to the transfer vector by means of an isocyanate group. In a preferred embodiment, the compound covalently bound to the matrix is bound to the transfer vector by means of a hydroxyl group.

The connecting complex can further comprise a molecule selected from the list comprising: sugar, peptide, lipid or any combination thereof and where said molecule is located between the gene transfer vector and the carboxyl group, amino group, isocyanate group and hydroxyl group of the compound covalently bound to the polymer matrix, this molecule is preferably located between the R₂ group of the compound of general formula (I) and the gene transfer vector. Therefore, the compound of general formula (I) and one of the molecules would comprise the connecting complex.

Therefore, in another preferred embodiment the connecting complex bound to the surface of the polymer matrix can bind to the transfer vector by means of a molecule selected from the list comprising: sugar, peptide, lipid or any of the combinations thereof.

The connecting complex of the bioactive surface of the invention can therefore comprise the compound containing a group selected from a carboxyl group, an amino group, an isocyanate group and a hydroxyl group anchored to the polymer matrix, preferably a PFM (pentafluorophenyl methacrylate) precursor, and at least one compound selected from the list comprising: a sugar, a peptide, a lipid or any combination thereof.

As used herein, the term "vector" or "gene transfer vector" refers to any system used for introducing an exogenous nucleic acid into a cell. The transfer vector must contain at least one reactive group through which it can react with the connecting complex, either with the compound anchored to the surface of the polymer matrix, or with another compound which can be part of the connecting complex, such as for example, but not limited to, a sugar, a peptide or a lipid. In a preferred embodiment, the gene transfer vector contains a reactive group through which it can react with the compound anchored to the polymer matrix, such as for example, but not limited to, an amino group or a carboxyl group. Therefore, in a preferred embodiment, the gene transfer vector of the bioactive surface of the invention comprises at least one amino group or one carboxyl group. For example, when the compound anchored, or covalently bound, to the surface of the polymer matrix is PFM, the gene transfer vector can react with the PFM if it contains an amino group. Therefore, in a more preferred embodiment the gene transfer vector of the bioactive surface of the invention comprises at least one amino group.

There are various viral and non-viral vectors that are known in the state of the art. Viral vectors suitable for putting the invention into practice include, but are not limited to the following: adenoviral vectors, adeno-associated vectors, retroviral vectors, lentiviral vectors, alpha viral vectors, herpes viral vectors, poxvirus-derived vectors and coronavirus-derived vectors. Non-viral vectors suitable for putting the invention into practice include, but are not limited to the following: polyamines, peptides, polypeptides, proteins, dendrimers, lipid/polyamine complexes and inorganic nanoparticles engineered for containing amino groups. The gene transfer vector of the bioactive surface of the invention can be a viral or non-viral vector. Therefore, in a preferred embodiment the gene transfer vector of the bioactive surface of the invention is selected from the list comprising: nucleic acid-polyamine complex, nucleic acid-peptide complex, nucleic acid-polypeptide complex, nucleic acid-protein complex, nucleic acid-dendrimer complex, nucleic acid-lipid-polyamine complex, nucleic acid-modified inorganic nanoparticle complex, adenoviral vector, adeno-associated vector, retroviral vector, lentiviral vector, alpha viral vector, herpes viral vector, poxvirus-derived vector or coronavirus-derived vector.

In a preferred embodiment, the gene transfer vector of the bioactive surface of the invention is a non-viral vector. In a more preferred embodiment, the non-viral gene transfer vector of the bioactive surface of the invention is a nucleoprotein complex, i.e., a complex formed by a nucleic acid and a peptide or polypeptide compound capable of facilitating the entry of the complex into the cell and of enhancing the biological function of the nucleic acid.

In another preferred embodiment, the gene transfer vector of the bioactive surface of the invention is a viral vector. In a more preferred embodiment, the viral gene transfer vector of the bioactive surface of the invention is a lentiviral vector, i.e., a lentiviral particle modified for reducing or eliminating its virulence, as well as for containing the nucleic acid to be transferred and for increasing the efficiency of said transfer. In another more preferred embodiment, the viral gene transfer vector of the bioactive surface of the invention is an adenoviral vector, i.e., an adenoviral particle modified for reducing or eliminating its virulence, as well as for containing the nucleic acid to be transferred and for increasing the efficiency of said transfer.

Therefore, in a preferred embodiment the bioactive surface of the invention comprises a polymer matrix and a gene transfer vector, preferably an adenoviral vector or a lentiviral vector, where said transfer vector is bound to the surface of said polymer matrix by means of a connecting complex comprising at least one PFM molecule bound to the surface of the polymer matrix of the invention.

The bioactive surface of the invention can be used either for completely or partially manufacturing an implantable device or for completely or partially coating an implantable device.

Therefore, a second aspect of the invention relates to an implantable device, hereinafter "implantable device of the invention", characterized in that at least one part of its surface is coated with the bioactive surface of the invention.

As used herein, the term "implantable device" refers to a device which can be applied to the surface of the body of an animal, preferably a human, or which can be inserted into the body of the animal or of the human.

In a preferred embodiment, the implantable device of the invention is selected from the list comprising: a stent, an orthopedic prosthesis, a biocompatible membrane, a porous polymer, a patch, a suture, a capsule and a particle.

A third aspect of the invention relates to the use of the bioactive surface of the invention or of the implantable device of the invention for transferring a nucleic acid to a cell contacting the bioactive surface of the invention.

As used herein, the term "nucleic acid" refers to a polymeric form of nucleotides of any length, both ribonucleotides and deoxyribonucleotides. Therefore, the nucleic acid can be a deoxyribonucleic acid, such as for example, but not limited to, double-stranded DNA, single-stranded DNA or circular DNA, or a ribonucleic acid, such as for example, interference RNA. The nucleic acid is preferably DNA, more preferably double-stranded DNA, and yet more preferably double-stranded circular DNA.

The bioactive surface of the invention or the implantable device of the invention can be used for transferring a nucleic acid to a prokaryotic or eukaryotic cell. In a preferred embodiment, the bioactive surface of the invention or the implantable device of the invention is used for transferring a nucleic acid, preferably a DNA, to a eukaryotic cell. In a more preferred embodiment, the bioactive surface of the invention or the implantable device of the invention is used for transferring a nucleic acid, preferably a DNA, to an animal cell. In an even more preferred embodiment, the bioactive surface of the invention or the implantable device of the invention is used for transferring a nucleic acid, preferably a DNA, to a human cell.

A fourth aspect of the invention relates to a method for transferring a nucleic acid to a cell (hereinafter, "first method of the invention") which comprises contacting the bioactive surface of the invention or the implantable device of the invention with said cell.

The first method of the invention can be used for transferring a nucleic acid to a prokaryotic or eukaryotic cell. In a preferred embodiment, the first method of the invention is a method for transferring a nucleic acid, preferably a DNA, to a eukaryotic cell. In a more preferred embodiment, the first method of the invention is a method for transferring a nucleic acid, preferably a DNA, to an animal cell. In an even more preferred embodiment, the first method of the invention is a method for transferring a nucleic acid, preferably a DNA, to a human cell.

A fifth aspect of the invention relates to a kit for carrying out the first method of the invention (hereinafter "first kit of the invention") comprising:
a) a polymer matrix,
b) a connecting complex comprising at least one compound covalently bound to the surface of the polymer matrix (a) containing a group selected from a carboxyl group, an amino group, an isocyanate group and a hydroxyl group; and
c) at least one element necessary for forming a gene transfer vector;
where the compound of complex (b) is covalently bound to the transfer vector by means of a group selected from a carboxyl group, an amino group, an isocyanate group and a hydroxyl group.

In a preferred embodiment, the polymer matrix of the first kit of the invention is selected from the list comprising: polystyrene, polycarbonate, poly(ethylene carbonate), polyethylene, polyolefins, poly(diol citrate), polyfumarate, polylactides, polycaprolactone, polyacrylamides, polysiloxanes, polyesters, polyamides, glycolic/lactic acid copolymers, polyurethane or any combination thereof. In a more preferred embodiment, the polymer matrix of the first kit of the invention comprises polystyrene.

In a preferred embodiment, the connecting complex comprises a compound which is selected from the list comprising:

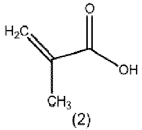

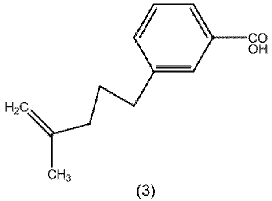

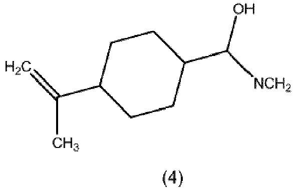

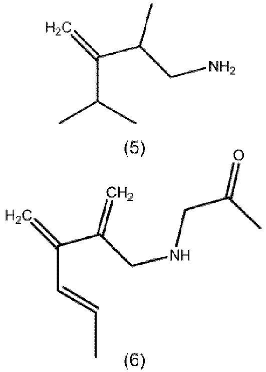

A sixth aspect of the invention relates to a kit for carrying out the first method of the invention (hereinafter "second kit of the invention") comprising:
a) the bioactive surface of the invention or the implantable device of the invention, and
b) at least one element necessary for forming a gene transfer vector.

The elements which are necessary for forming a gene transfer vector depend on the type of gene transfer vector and are known in the state of the art.

In a preferred embodiment, the first or second kit of the invention comprises at least one element necessary for forming a gene transfer vector selected from the list comprising: polyamine, peptide, polypeptide, protein, dendrimer, lipid-polyamine, modified inorganic nanoparticle and plasmid encoding a capsid/envelope protein of a viral vector. Preferably, said viral vector is selected from the list comprising adenoviral vector, adeno-associated vector, retroviral vector, lentiviral vector, alpha viral vector, herpes viral vector, poxvirus-derived vector or coronavirus-derived vector.

In a preferred embodiment, the first or second kit of the invention comprises at least one element necessary for forming a nucleopeptide complex. Examples of elements necessary for forming a nucleopeptide complex include, but are not limited to, polylysine, fusogenic peptides, fusogenic proteins or lipid-peptide complexes.

In a preferred embodiment, the first or second kit of the invention comprises at least one element necessary for forming a lentiviral vector. Examples of elements necessary for forming a lentiviral vector include, but are not limited to, plasmids encoding capsid proteins of HIV, FIV, or EIAV; plasmids encoding viral envelopes having reduced toxicity and/or immunogenicity, and with tropism suitable for the cell type to be modified, such as for example, but not limited to, VSV-G, RD114, envelope proteins of LCMV, RRV, filovirus, lysovirus, or rabies virus; plasmids encoding other lentiviral proteins, including those which confer lentiviral particles with a replicative and non-replicative nature, and also those which confer them with an integrative or non-integrative nature.

In a preferred embodiment, the first or second kit of the invention comprises at least one element necessary for forming an adenoviral vector. Examples of elements necessary for forming an adenoviral vector include for example, but are not limited to, plasmids encoding capsid proteins of type 2 or type 5 adenovirus. Said proteins could be modified for increasing or limiting their tropism or for reducing their immunogenicity and/or toxicity.

A seventh aspect of the invention relates to a method for preparing the bioactive surface of the invention (hereinafter, "second method of the invention"), which comprises contacting:
- a polymer matrix comprising a connecting complex covalently bound to its surface by means of a compound containing a group selected from a carboxyl group, an amino group, an isocyanate group and a hydroxyl group and
- a gene transfer vector,
in conditions which allow the gene transfer vector to bind to said connecting complex. Said connecting complex preferably comprises at least one compound of general formula (I) described above, and more preferably a PFM precursor anchored to the surface of the polymer matrix.

In a preferred embodiment, the second method of the invention comprises contacting the polymer matrix having the connecting complex bound to its surface with the gene transfer vector at a temperature between 20 and 40°C for a time period between 5 minutes and 24 hours.

In a more preferred embodiment, the second method of the invention comprises contacting the polymer matrix having the connecting complex bound to its surface with the gene transfer vector at a temperature between 30 and 37°C for a time period between 30 and 60 minutes.

In a more preferred embodiment, to favor the binding of the gene transfer vector to the polymer matrix having the connecting complex bound to its surface, the gene transfer vector on the polymer matrix having the connecting complex bound to its surface is centrifuged.

Said centrifugation is preferably performed at a speed between 100 and 20,000 Xg, and more preferably at a speed between 1,000 and 3,000 Xg. Said centrifugation is preferably performed at a temperature between 20 and 40°C and for a time period between 5 minutes and 24 hours. More preferably, said centrifugation is performed at a temperature between 30 and 37°C and for a time period between 30 and 60 minutes.

In another more preferred embodiment, to favor the binding of the gene transfer vector to the polymer matrix having the connecting complex bound to its surface, the matrix having the connecting complex bound to its surface is subjected to a negative pressure. Said pressure is preferably between -10⁻⁴ and -10⁻¹ mBar, and more preferably between -9.10⁻³ and -9.10⁻² mBar. Said negative pressure is preferably exerted at a temperature between 20 and 40°C and for a time period between 5 minutes and 24 hours. More preferably, said negative pressure is exerted at a temperature between 30 and 37°C and for a time period between 30 and 60 minutes.

In another more preferred embodiment, to favor the binding of the gene transfer vector to the polymer matrix having the connecting complex bound to its surface, the matrix is subjected to a combination of centrifugation and negative pressure. Said pressure is preferably between -10⁻⁴ and -10⁻¹ mBar, and more preferably between -9.10⁻³ and -9.10⁻² mBar. Said centrifugation is preferably performed at a speed between 1,000 and 20,000 Xg, and more preferably at a speed between 1,000 and 3,000 Xg. This combination is exerted at a temperature between 20 and 40°C and for a time period between 5 minutes and 24 hours. More preferably, said combination is applied at a temperature between 30 and 37°C and for a time period between 30 and 60 minutes.

In a preferred embodiment of the second method of the invention, the polymer matrix having the connecting complex bound to its surface is obtained by contacting said connecting complex with said polymer matrix under conditions which allow the polymer matrix to bind covalently to the compound of the connecting complex. In a preferred embodiment, the polymer matrix having the connecting complex bound to its surface is obtained by means of a method selected from the list comprising: chemical deposition, polymerization, plasma polymerization and iCVD (initiated chemical vapor deposition).

In a preferred embodiment of the second method of the invention, the polymer matrix having the connecting complex bound to its surface comprising at least one compound containing a group selected from a carboxyl group, an amino group, an isocyanate group and a hydroxyl group, preferably a PFM, is obtained by:
a) activating the polymer matrix by means of cold plasma, and
b) passing a stream of connecting complex comprising at least one compound covalently bound to the surface of the polymer matrix obtained from step (a) containing a group selected from a carboxyl group, an amino group, an isocyanate group and a hydroxyl group, preferably a PFM.

In another preferred embodiment, the connecting complex comprises a compound which is selected from the list comprising compound (2) to (6) described above:

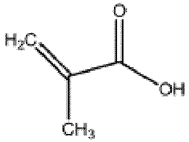

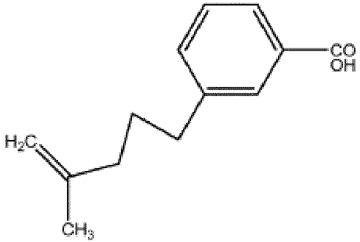

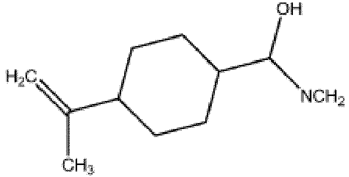

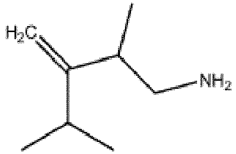

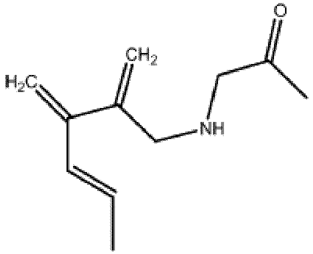

According to another preferred embodiment, the connecting complex further comprises a molecule selected from the list comprising sugar, peptide, lipid or any combination thereof, where said molecule can bind to the compound containing the connecting complex before or after it covalently binds to the polymer matrix.

Throughout the description and the claims, the word "comprises" and its variants do not intend to exclude other technical features, additives, components or steps. For the persons skilled in the art, other objects, advantages and features of the invention will be inferred in part from the description and in part from the practice of the invention. The following drawings and examples are provided by way of illustration and do not intend to limit the present invention.

### Description of the Drawings

Figure 1 shows the reaction of the amino group with PFM.
Figure 2 (a) shows the structure of a lentiviral particle, (b) the structure of the extra-membranous domain of VSV-g, the envelope protein of pseudotyped lentiviral particles, which will react with PFM.
Figure 3 shows a fluorescence microphotograph of cell culture (TC) dishes that are not treated (a) and treated with PFM (b).

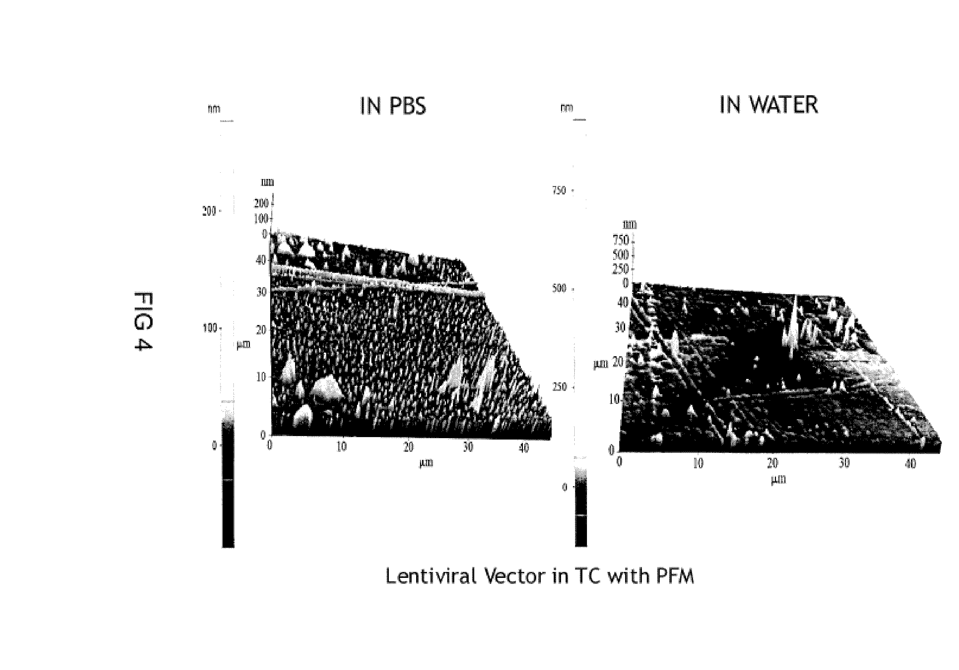

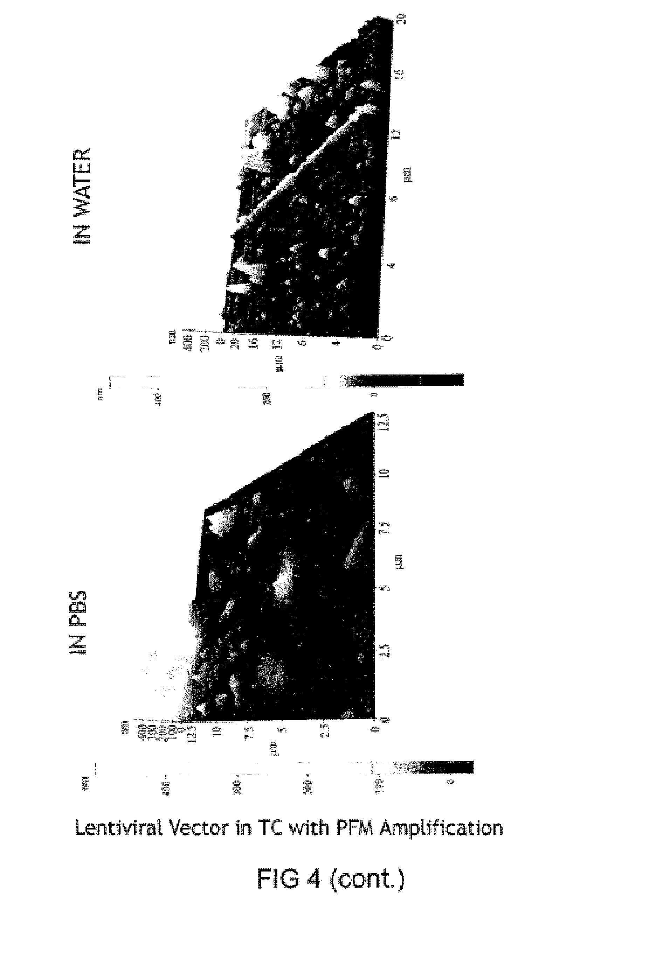

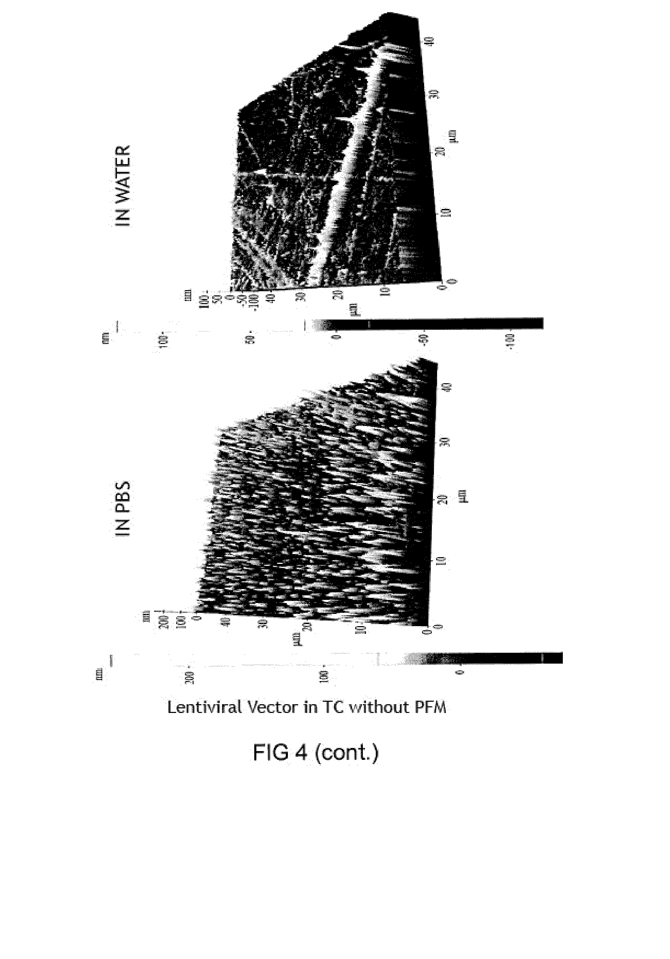

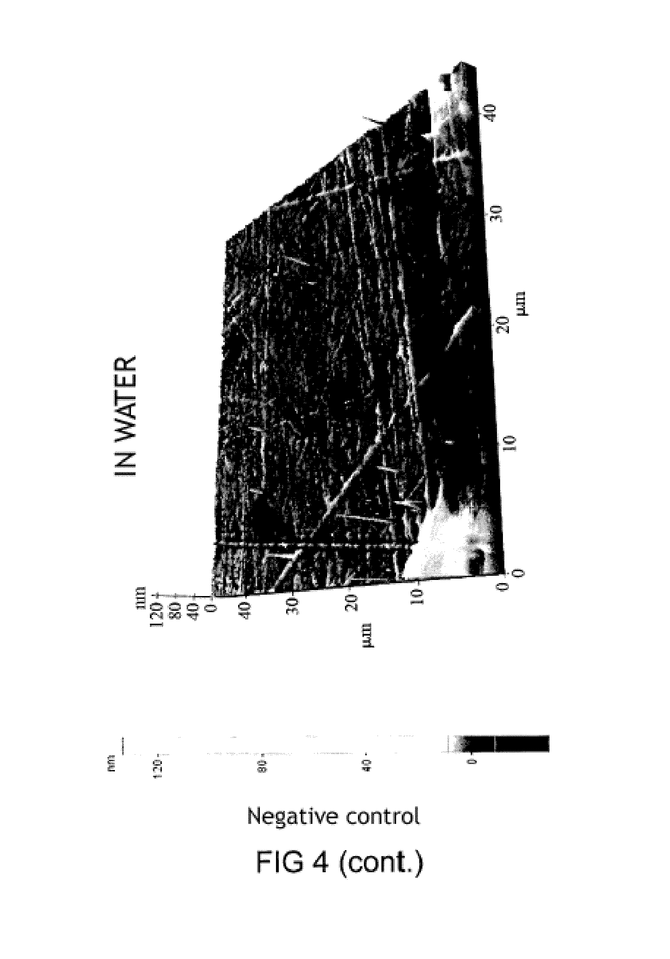

Figure 4 shows the AFM images of the lentiviral particles deposited on TC dishes after a 30- minute incubation. Two different washings were performed, one with PBS, where the salts are seen, and the other with distilled water. The negative control is the TC dish which is not modified.
Figure 5 shows the AFM images of the lentiviral particles deposited on TC dishes which are modified or not modified with PFM after a 3-hour incubation. The washing was done with distilled water.
Figure 6 shows the AFM images of the lentiviral particles deposited on Petri dishes (PD) after a 3-hour incubation. The washing was done with distilled water.
Figure 7 shows the microphotographs of the expression of green fluorescent protein (GFP) in human cells (HEK-293) grown on TC dishes coated with lentiviral particles (encoding GFP) by means of the method of the invention in the presence and in the absence of centrifugation. The incubation time was 60 minutes.
Figure 8 shows the analysis by means of flow cytometry of the cells of Figure 7. The positive control corresponds to cells infected using the conventional method (incubation with lentiviral particles in suspension).
Figure 9a) shows dishes coated with Amino-PEG. b) dishes coated with lentivirus.
Figure 10 shows the transduction of human cells by means of lentiviral particles deposited on different surfaces. The control corresponds to cells transduced by means of the conventional method.
Figure 11 shows the lentiviral transduction of human cells in a TC dish, half of which is treated with PFM and the other half is not treated with PFM, with the corresponding image obtained with AFM.
Figure 12 shows the transduction of cells by adenoviral particles and DNA-lipofectamine complexes deposited on TC dishes by means of the method of the invention.
Figure 13 shows the percentage of siRNA transfection using the RAWA-H8 peptide with the surfaces of the invention.
Figure 14 shows the transduction activity of the dishes modified with CRS technology after one month of storage at -80°C.
Figure 15 shows the production of iPS cell colonies by means of the conventional method (+ control) and by using the technology of the invention (CRS).

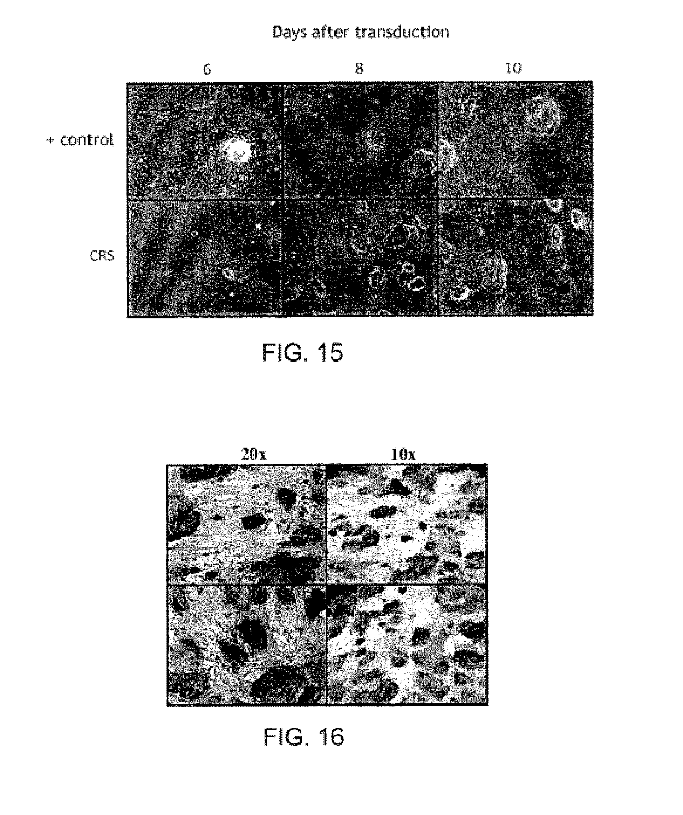

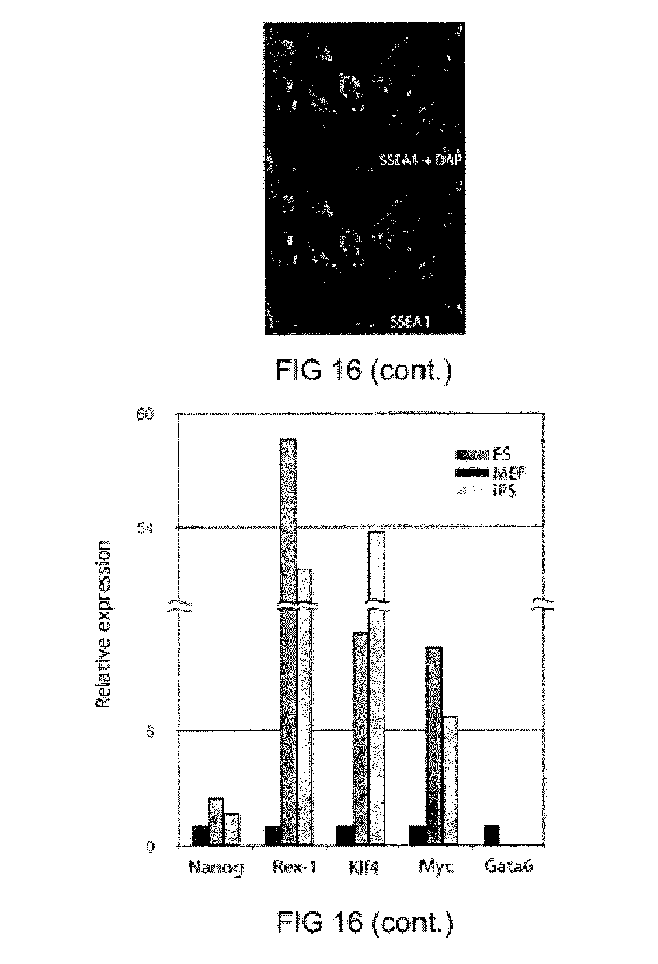

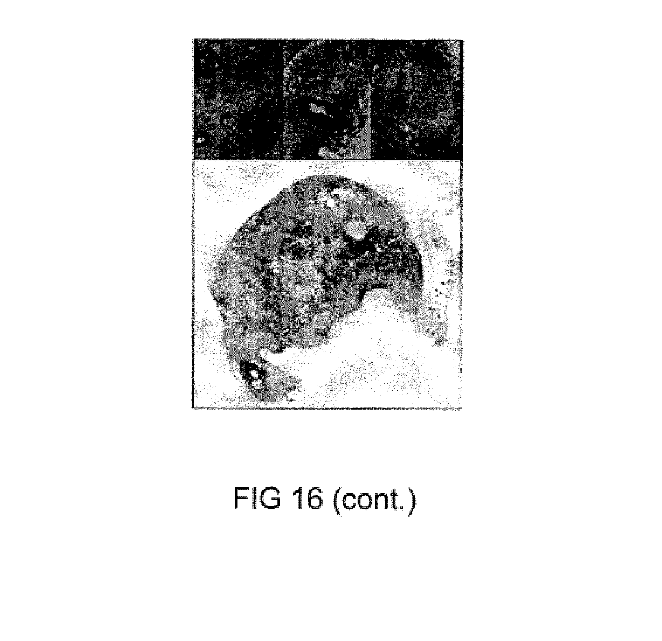

Figure 16 shows the characterization of murine iPS cells obtained by means of the technology of the invention. A, staining with alkaline phosphatase. B, staining with anti_SSEA-1 antibody ; C, analysis of the expression of various pluripotency marker genes (Gata6 is a negative marker) by means of quantitative RT-PCR in the obtained iPS cells, compared with ES cells (ES) and embryonic fibroblasts (MEF) from the same strain of mouse; D, teratocarcinomas containing cells from the three embryonic layers formed in immunodeficient mice four weeks after the subcutaneous injection of 2x10⁶ cells/animal.

### Examples

The following specific examples which are provided in this patent document serve to illustrate the nature of the present invention. These examples are only included for illustration purposes and must not be interpreted as limiting the invention claimed herein. Therefore, the examples described below illustrate the invention without limiting the field of application thereof.

### Example 1: Gene transfer with lentiviral vectors

Polystyrene was used as polymer matrix; specifically two types of easily available culture dishes were used: untreated polystyrene Petri dishes (PD) and cell culture (Tissue Culture, TC) dishes.

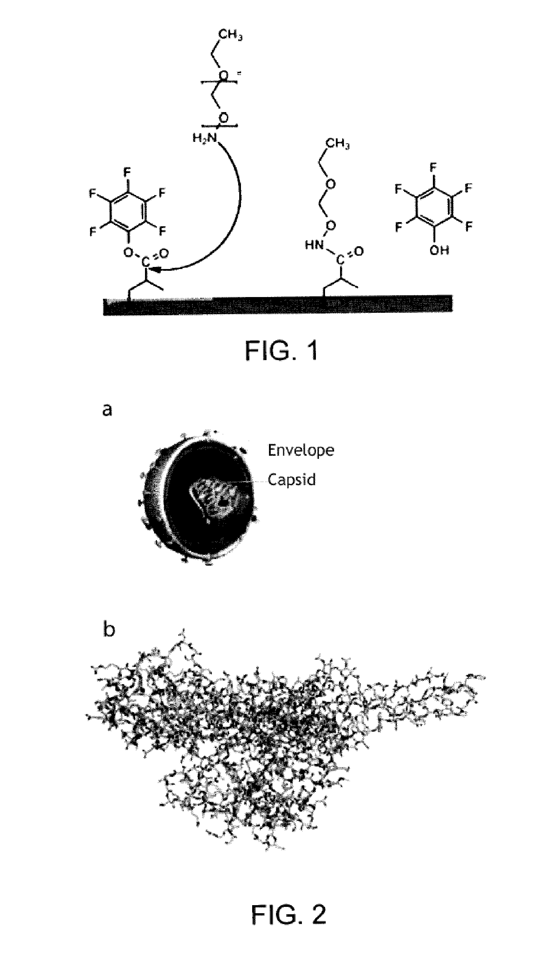

The surface of the dishes was treated with cold plasma for introducing pentafluorophenyl methacrylate (PFM) by means of grafting. The dishes treated with PFM were already prepared for reacting with a gene expression vector that had an amino group (Figure 1).

Given the protein nature of the ineffective particles (lentivirus, adenovirus, etc.), they have a high content of amino groups, so they can react with the treated surface.

For example, in the case of lentiviral particles (Figure 2a), the adhesion to the surface occurs through the envelope protein, specifically the glycoprotein of vesicular stomatitis virus (VSV-g, Figure 2b), which has a large amount of free amino groups on which a reaction can occur.

Atomic force microscopy (AFM) was used to view the surface particles whereby the difference of the particles adhered to surfaces treated with PFM and to untreated surfaces can be seen. In the case of transduction efficiency, it was analyzed by means of introducing a reporter gene encoding green fluorescent protein (GFP) the expression of which can be detected by fluorescence microscopy and cytometry (cytometry also allowed quantifying transduction efficiency).

### 1.1 Coating the polymer matrix with PFM

### Objective

To obtain polystyrene dishes chemically modified by means of PFM binding.

### Method

Polystyrene was used as polymer matrix; specifically two types of easily available culture dishes were used: untreated polystyrene Petri dishes (PD) and commercial cell culture (Tissue Culture, TC) dishes.

The dishes were exposed to cold Ar plasma for introducing the PFM by means of grafting. The method followed was the following:
1. The culture dishes (TC or PD) were introduced in the plasma reactor.
2. Vacuum was applied in the reactor until a pressure of about 6x10⁻³ mBar. Once this pressure was reached, an Ar plasma with a power of 40w was applied for 5 minutes.
3. Once this time lapsed, a stream of PFM was passed through for 15 minutes for introducing it on the surface of the dishes.

To confirm that the dishes were well-coated with PFM, they were reacted with fluorescein isothiocyanate (FTSC) (which can be viewed by means of fluorescence microscopy). This was performed by means of a micro-contact printing assay consisting of using two buffers made of polydimethylsiloxane (PDMS) with the same logotype (IQS/CNM) that are previously submerged in a 0.5 mM FTSC solution for 1 hour. The buffers were gently deposited on the dishes, one on a dish treated with PFM and the other on a dish without PFM.

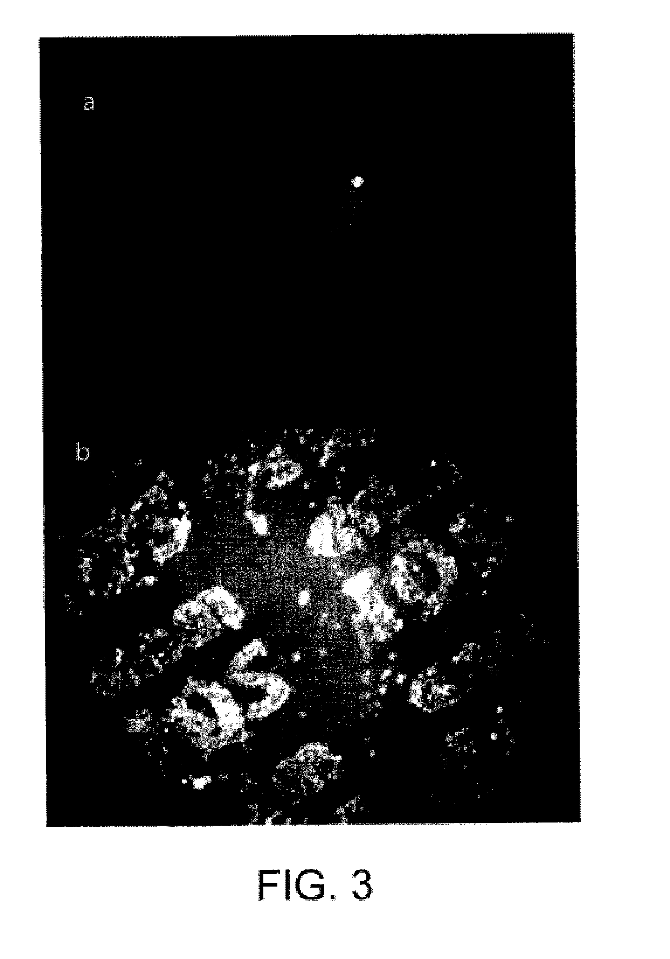

After washing with water for 30 minutes, the dishes were observed under a fluorescence microscope. The dishes treated with PFM clearly showed the IQS/CNM drawing formed by the FTSC molecules (Figure 3), which confirmed that the PD and TC dishes were coated with PFM and that the PFM reacted with the FTSC.

### 1.2. Atomic force microscopy (AFM)

### Objective

To view the ineffective particles on culture surfaces through AFM, to enable evaluating the efficiency of different treatments.

### Method

PD and TC dishes were used with the following treatments:
- Half the dishes with PFM/the other half without PFM.
- Entire dishes with PFM.
- Entire dishes without PFM.

All the dishes were treated in a similar manner, varying the exposure time of the viral particles. The method was the following:
- for each dish, a dilution of lentiviral particles in PBS was prepared with a titer of 10⁷ transduction units (TU)/ml, in a volume of 1 ml.
- the particle suspension was added on the dishes and was incubated at room temperature for different times, with orbital stirring (30 rpm).
- the medium was removed and washed 5 times with PBS or 3 times with milliQ water.
- The edges of the dishes were cut to enable introducing the dishes in the AFM.
- Finally, Ar was passed over the surface to eliminate the remaining non-adhered powder and particles.

The following experiments were performed:
- Half the dishes with PFM/the other half without PFM:
   ○ Lentivirus, 30 minutes of exposure with orbital stirring.
- Entire dishes with PFM:
   ○ Lentivirus, 3 hours of exposure with orbital stirring.

### Results

### Experiment 1: TC dishes with lentivirus x 30 minutes

It can be seen in all cases that the surface with PFM contains what seems to be lentiviral particles given their size (150-200 nm). If a thorough observation is made, height up to 400 nm can be seen in some areas, which may indicate particle aggregation in these areas. In contrast, in the part without PFM, there are practically no particles greater than 30 nm.

### Experiment 2: TC dishes with lentivirus x 3 hours

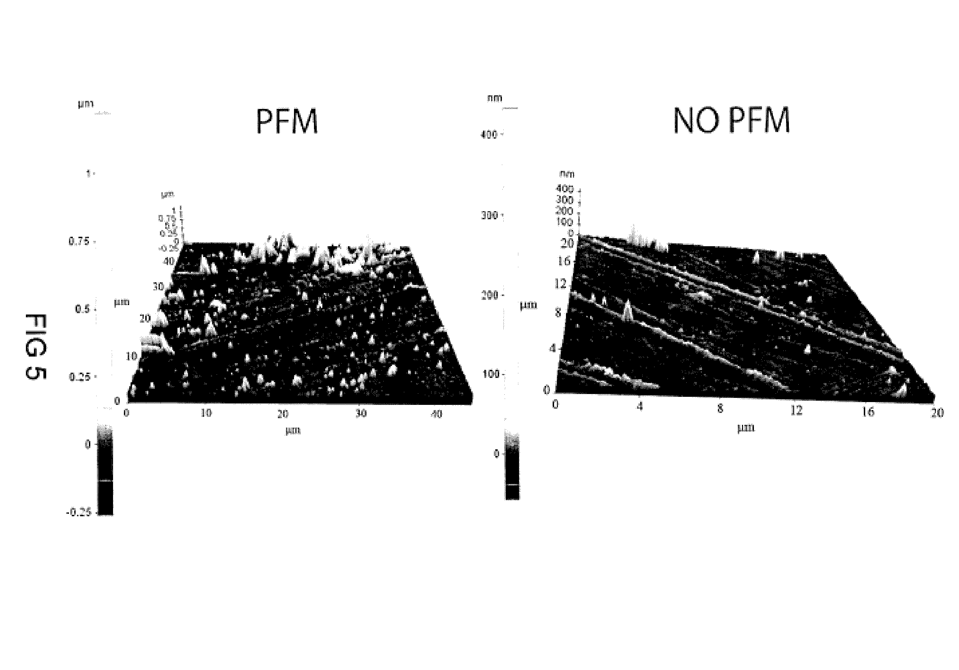

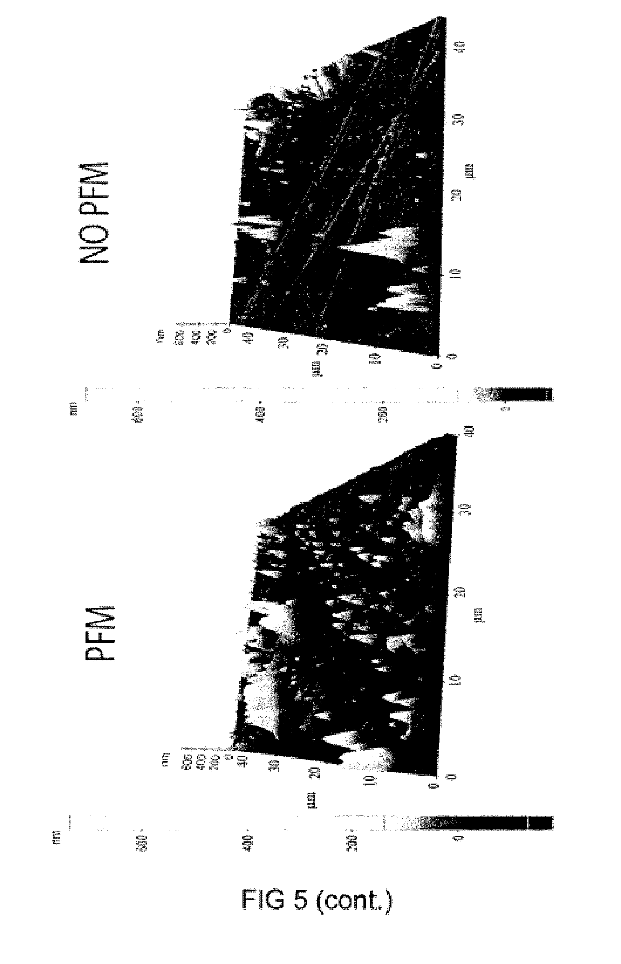

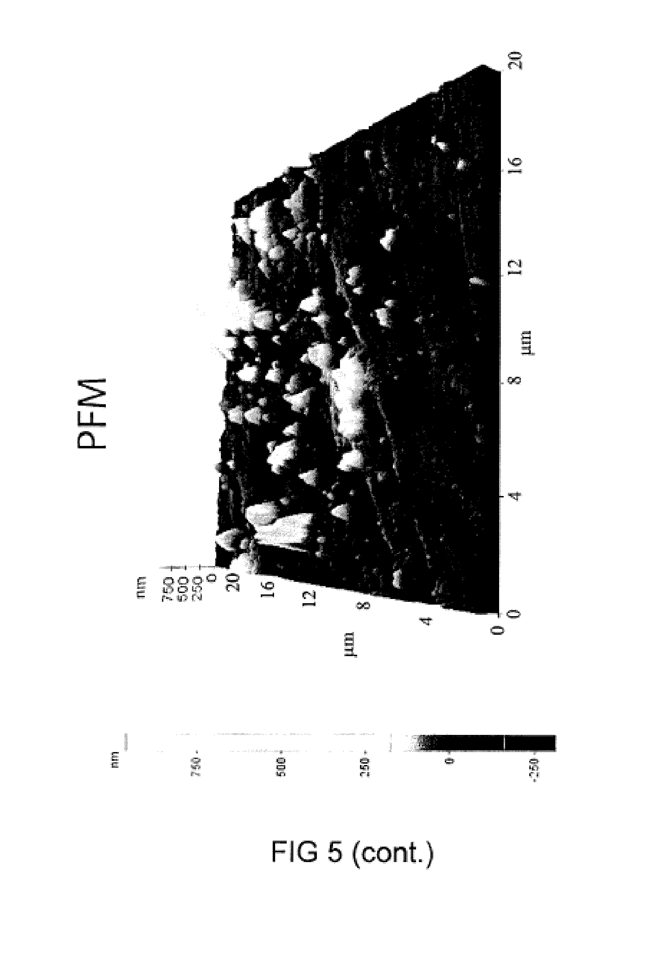

The results (Figure 5) demonstrate a significant increase in the amount of surface particles with respect to those of Experiment 1. The size of the particles is also greater in some cases, which can indicate a higher degree of particle aggregation. A greater particle distribution is observed upon zooming in. In the case of negative controls, few particles are observed, which indicates a certain affinity for the hydrophilic surface.

### Experiment 3: PD dishes with lentivirus x 3 hours

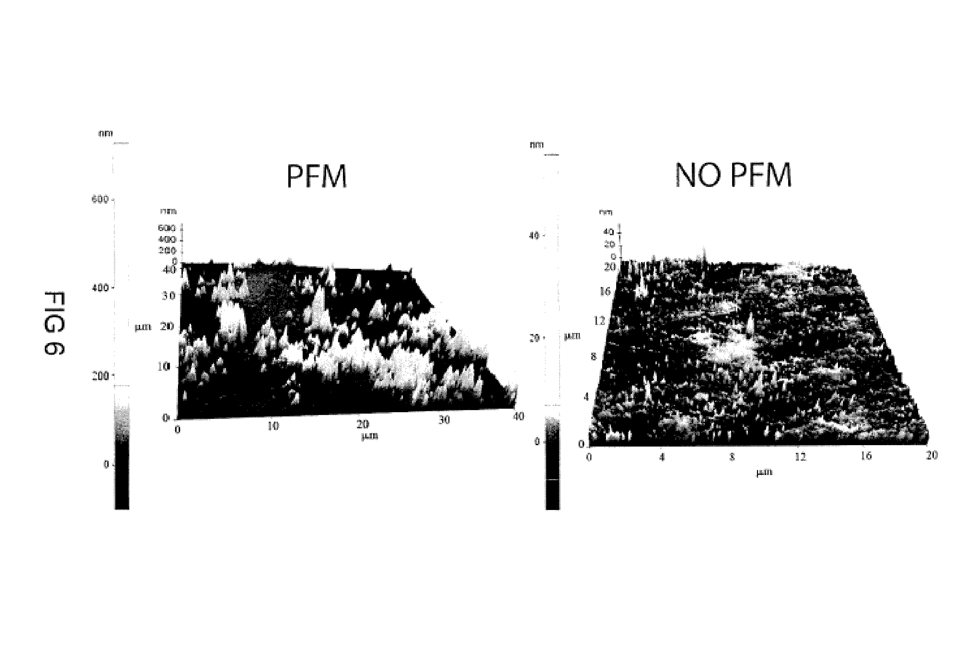

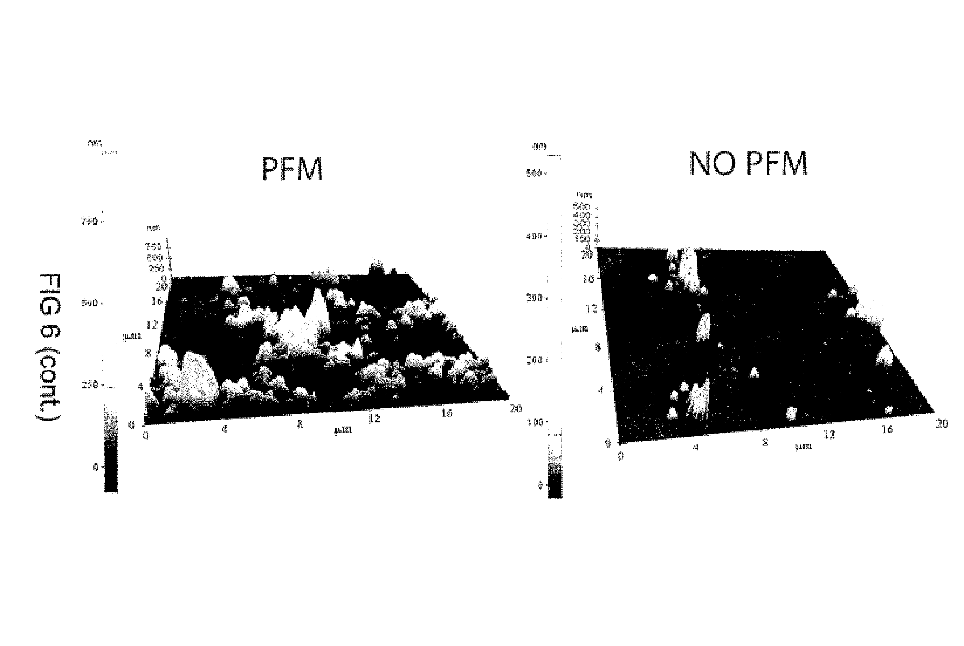

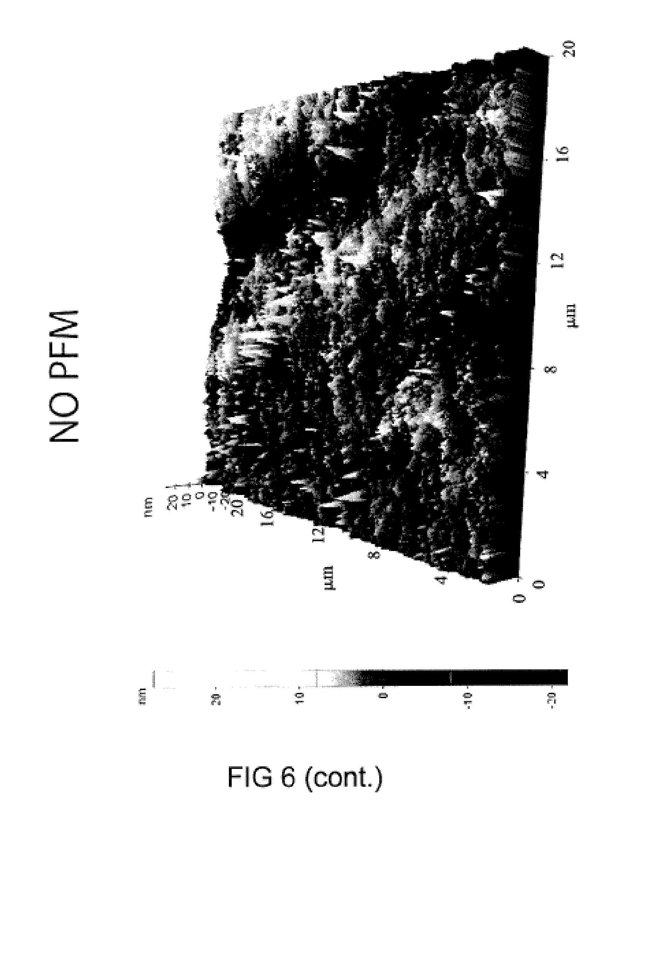

In this case, a great difference is observed between one surface and another (Figure 6). The amount of particles adhered to the surface is high with respect to the preceding experiments. If a comparison is made, very clear differences are seen between the dishes with PFM and the dishes without PFM. Like the preceding experiments, there seems to be significant particle aggregation. Unlike the preceding experiments, the surface without PFM is hydrophobic, so the proteins do not tend to adhere to same by hydrophilic interactions.

### 1.3. In vitro genetic modification of human cells with lentiviral vectors

### • Experiment 1

### Objective

This first experiment was intended for observing the difference between a TC dish and a TC dish treated with PFM. Furthermore, it was also intended for improving the diffusion of lentiviral particles so that they can react with the PFM by means of centrifugation.

### Method

The experiments performed were the following:

| | PFM | No PFM |
|---|---|---|
| TC | Sample 1 | ----- |
| Centrifuged PD | Sample 2 | ----- |
| TC | ----- | Sample 3 |

The materials used were
o Cells: (HEK-293T 4x10⁵ cells/dish)
o Stock of lentiviral particles: LV-ZS Green/ApoCi sh4 (2x10⁷ TU/ml)

The protocol used was the following:
o The lentiviral particles (500 µL from a 1:3 stock dilution) were added to the dish and incubated for 30 minutes in the incubator at 37°C or for 60 minutes in the centrifuge at 3000 Xg, where appropriate.
○ It was washed with a cell culture medium (DMEM/10% FBS) X 5 times.
○ It was washed with 400 ml of culture medium for 10 minutes with orbital stirring.
○ The cells were seeded and the dish was introduced in the incubator.
○ After 48 hours, the cells were observed under fluorescence microscope and analyzed by means of flow cytometry to detect the expression of GFP.

### Results

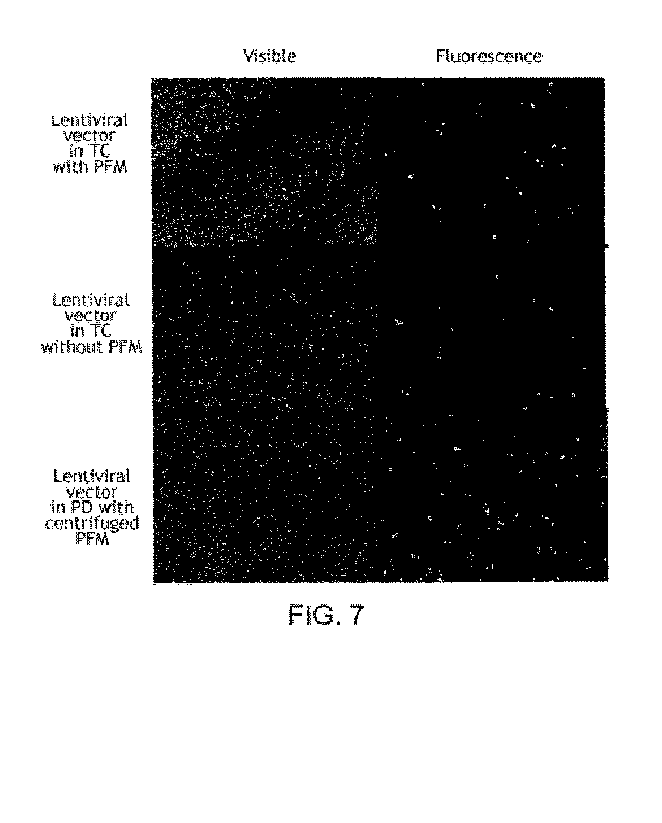

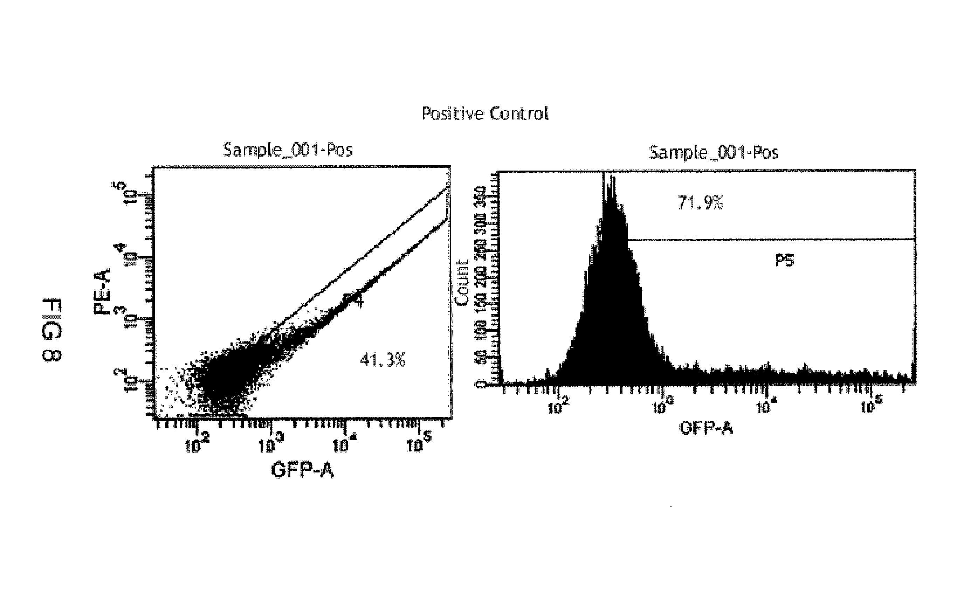

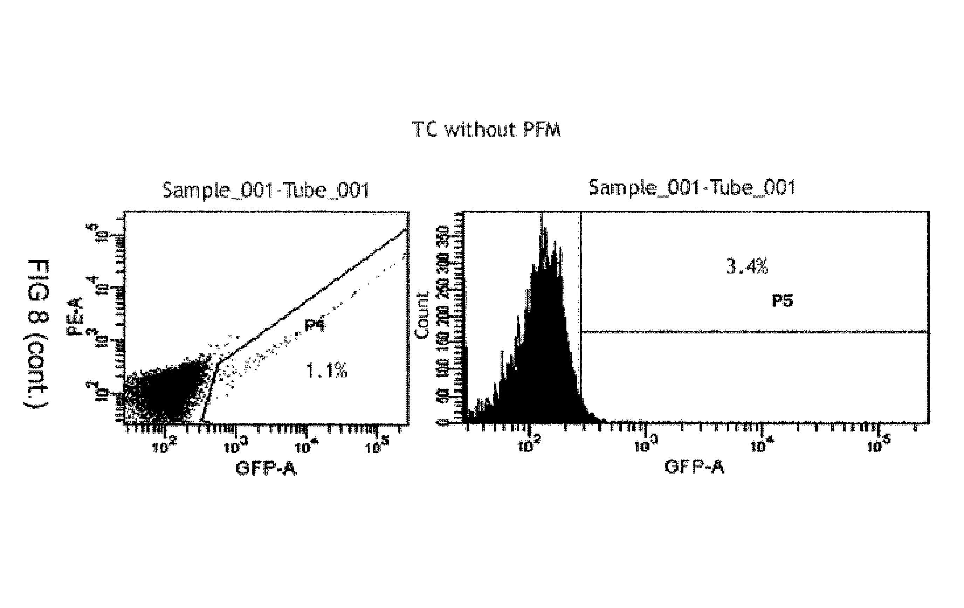

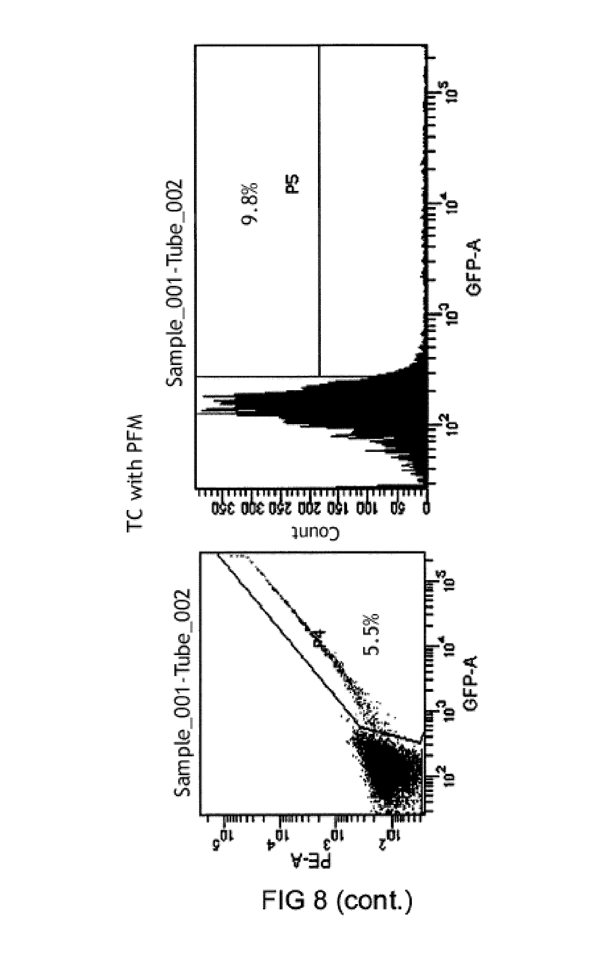

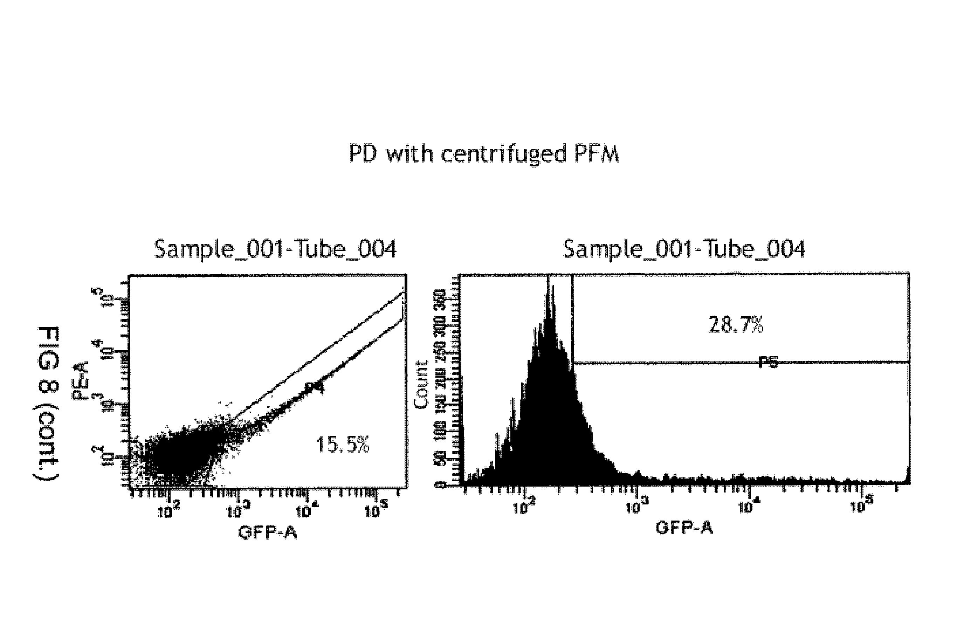

The results show a great difference between the dishes treated with PFM and the untreated dishes (Figure 7). Since the level of transduction is quantified by means of flow cytometry (Figure 8), the dishes treated with PFM without centrifugation are 4 times more effective than the untreated dishes, whereas in the treated dishes with centrifugation the level of transduction was of the order of 15 times more. These results demonstrate that the efficacy obtained with the dishes treated with PFM is much greater than the residual activity due to the non-specific, non-covalent binding of the particles to the hydrophilic surface.

### • Experiment 2

### Objective

To evaluate the efficiency of gene transfer in dishes treated with PFM in comparison with surfaces of similar hydrophobicity with respect to those that are treated with PFM, but not reactive.

### Method

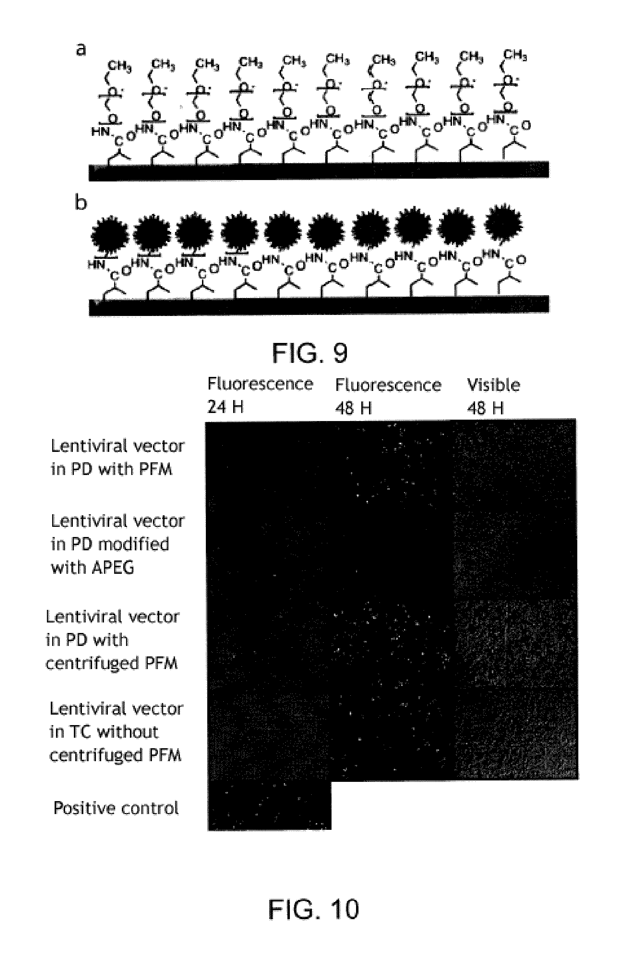

The following surfaces were used (Figure 9):
o Treated with PFM and blocked with amino-PEG (APEG).
○ TC dishes not treated with PFM.
o Dishes with PFM coated with virus

The experiments performed were the following:

| | PFM | No PFM |
|---|---|---|
| PD | Sample 1 | ----- |
| PD-APEG | Sample 2 | ----- |
| Centrifuged (PD) | Sample 3 | ----- |
| TC | ----- | Sample 5 |

The materials used were:
o Cells that were used: HEK-293 (4x10⁵ cells/dish).
○ Stock of lentiviral particles: LV-ZSGreen/ApoCi sh4 (2x10⁷ TU/ml).

Before covering with the lentivirus, the dishes which will be used as negative controls were blocked. To that end, 1 ml of a 10 mM amino-PEG solution was added on a PD dish coated with PFM, and incubated overnight with orbital stirring. It was washed with milliQ water the next morning. To cover with the lentivirus, the same protocol described in Experiment 1 was followed, although in this case the cells were not analyzed by means of cytometry.

### Results

The obtained results (Figure 10) showed once again that the dishes treated with PFM are capable of transducing a greater mayor of cells than the dishes which have not been treated with said molecule. Furthermore, this is clearly demonstrated when comparing the PD dishes with PFM with the APEG dishes, in which practically no expression of GFP is detected. In the case of centrifugation, a great increase in transduction in PD PFM dishes was also observed with respect to the TC dishes used for centrifugation.

### • Experiment 3

### Objective

To demonstrate the selective genetic modification of human cells *in vitro* by means of contact with surfaces only partially treated with the method of the invention.

### Method

A TC dish was covered, half with PFM and the other half without PFM, and the experiment was conducted with the conventional protocol (viral suspension for 30 minutes at 37°C).

### Results

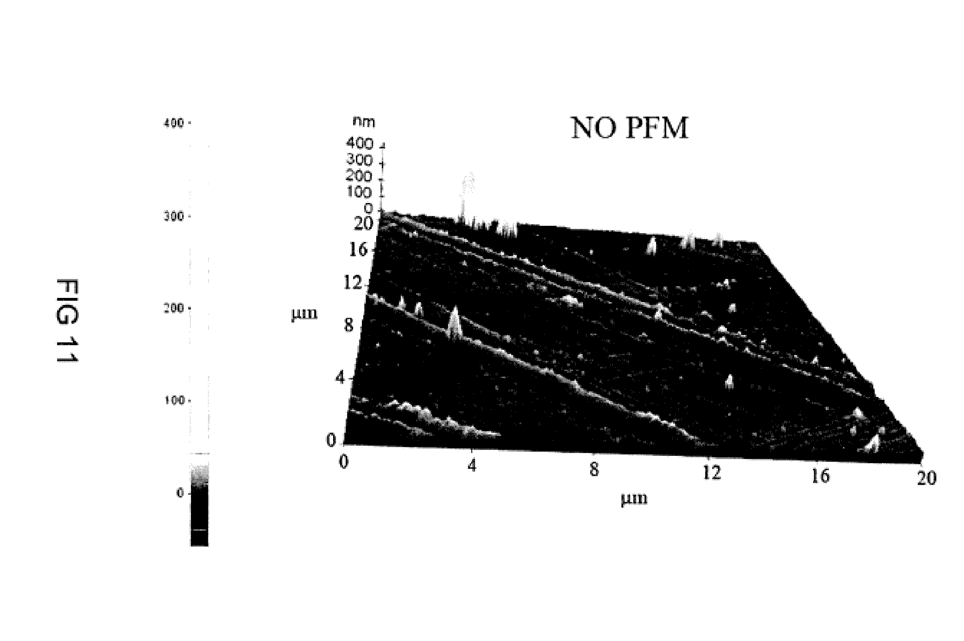

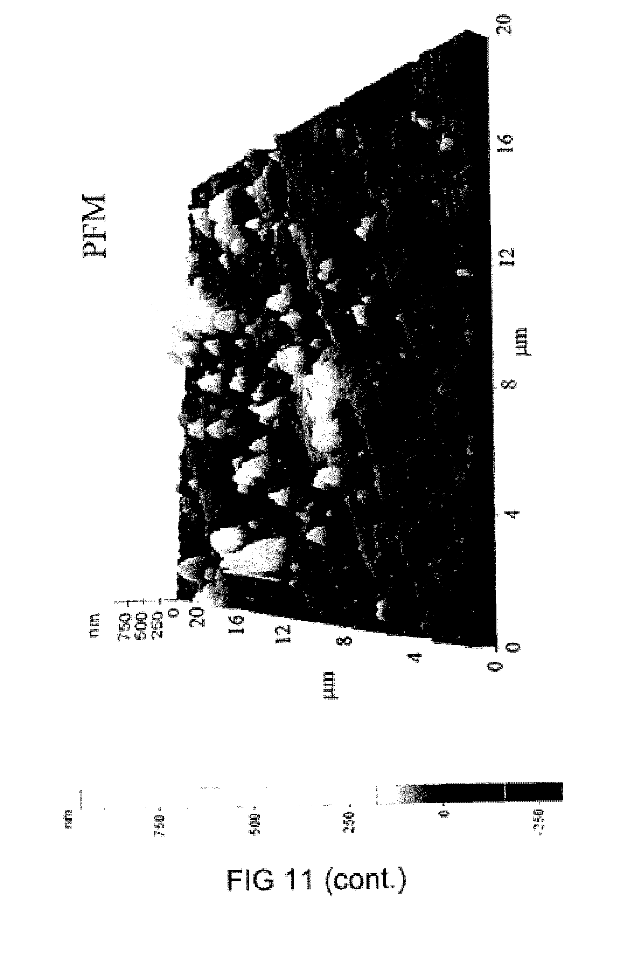

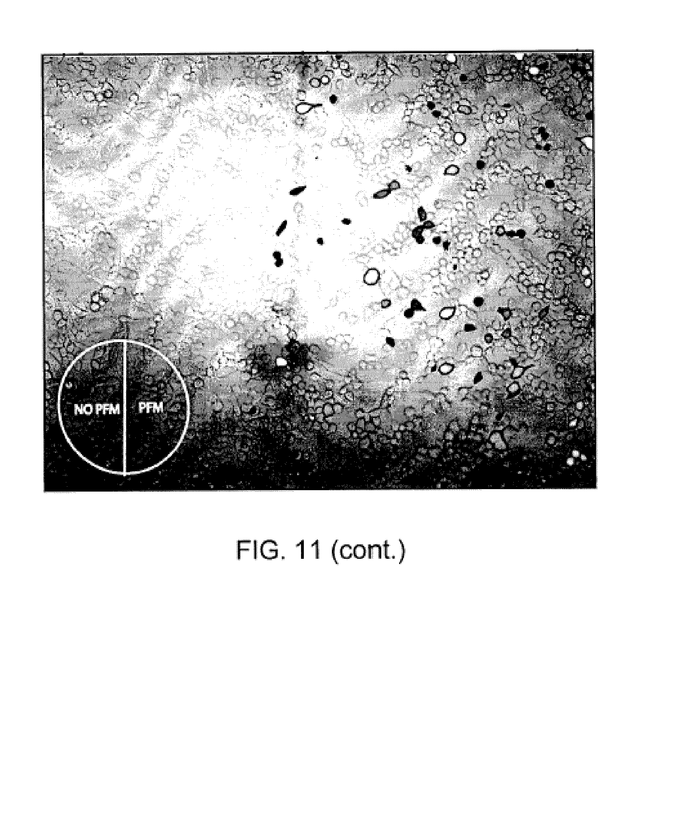

Figure 11 shows that the cells adhered to the surface where there was PFM selectively expressed the reporter gene (GFP), whereas the cells adhered to the part where there was no PFM were not transduced in a significant proportion.

### Example 2: Gene transfer using adenoviral particles

### Objective

To evaluate the efficacy of gene transfer in dishes treated with PFM by means of infection with adenovirus.

### Method

The materials used were:
o Cells: Hek-293T (4x10⁵ cells/dish)
o Stock of adenoviral particles: AdGFP (2x10⁹ TU/ml)

The protocol used was the following:
o The adenoviral particles (500 µL of a 1:3 stock dilution) were added to the dish and incubated for 30 minutes in the incubator at 37°C.
o It was washed with a cell culture medium (DMEM/10% FBS) X 5 times.
○ It was washed with 400 ml of culture medium for 10 minutes with orbital stirring.
○ The cells were seeded and the dish was introduced in the incubator.
○ After 48 hours, the cells were observed under fluorescence microscope.

### Results

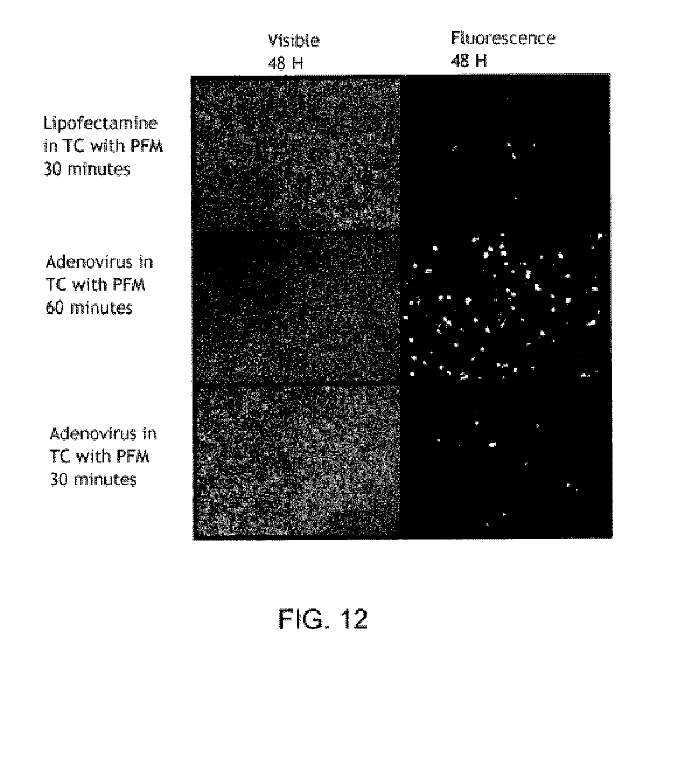

The results (Figure 12) show that genetic modification of the cells occurred since a high percentage of GFP expression can be observed in the cells under fluorescence microscope.

### Example 3: Gene transfer using DNA-lipofectamine complexes

### Objective

To evaluate the efficacy of gene transfer in dishes treated with PFM by means of transfection with the commercial transfection agent, lipofectamine (Lipofectamine™, Invitrogen).

### Method

The materials used were:
o Cells: HEK-293T 4x10⁵ (cells/dish)
o Lipofectamine™ 2000 (Invitrogen), prepared and at concentrations indicated by the manufacturer.

The protocol used was the following:
o The complexes with lipofectamine (500 µL of a 1:3 stock dilution) were added to the dish and incubated for 30 minutes in the incubator at 37°C.
o It was washed with a cell culture medium (DMEM/10% FBS) X 5 times.
○ It was washed with 400 ml of culture medium for 10 minutes with orbital stirring.
○ The cells were seeded and the dish was introduced in the incubator.
○ After 48 hours, the cells were observed under fluorescence microscope.

### Results

The results (Figure 12) show cells infected by means of this method. GFP expression is observed through fluorescence microscope.

### Example 4: siRNA transfer using RAWA-H8 peptide

### Objective

To evaluate the efficiency of the transfer of siRNA to human cells by means of the surfaces of the invention, using RAWA-H8 peptide as a transfection agent (Barajas R. Development and Evaluation of RAWA-H8 Model as a Highly Efficient Peptide Transfection System [Desarrollo y evaluación del modelo RAWA-H8 como sistema peptídico de transfección de alta eficiencia], Doctoral Thesis, 2006).

### Method

The materials used were:
- 30 mm Petri dishes treated with PFM (PD)
- Cells: HEK-293T (4x10⁵ cells/dish)
- RAWA-H8 peptide
- siRNA labeled with Alexa Fluor 555

The protocol used was the following:
1. A mixture containing 50 pmol of siRNA in DMEM with 10 mM HEPES was prepared in a total volume of 200 ml per dish (mixture 1).
2. A mixture containing 4.7 mg of RAWA-H8 peptide in DMEM with 10 mM HEPES was prepared in a total volume of 100 ml per dish (mixture 2).
3. Mixture 2 was added to mixture 1.
4. It was incubated for 15 minutes at 50°C.
5. The resulting sample contains the siRNA:RAWA-H8 transfection complexes. As the positive control of the experiment, a 1:2.3 (complex:medium) dilution of these complexes in a culture medium was added (1.5 ml/dish) to dishes with HEK-293 cells seeded the previous day (5x10⁵ cells/dish).
6. With the PDs already prepared according to the protocol described in preceding examples, different dilutions of the transfection complexes in culture medium (1.5 ml/dish) were added and the dishes were centrifuged for one hour at 3,000 g and 37°C.
7. The medium was removed and the dishes (5x50ml) were washed with culture medium.
8. 5x10⁵ HEK-293T cells were seeded in each of the dishes.
9. 6 hours after adding the positive control to the cells (step 5), the medium was changed.
10. The cells were collected after 24 hours and analyzed by means of flow cytometry to detect labeling with Alexa Fluor 555.

### Results

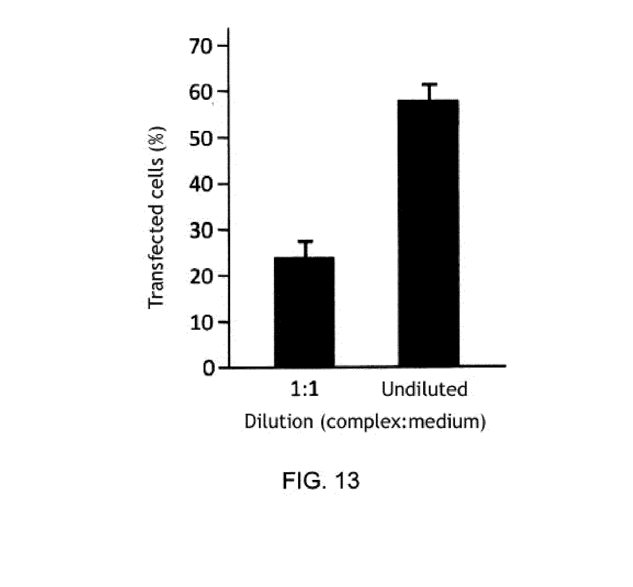

The results of the cytometry (Figure 13) showed a transfection efficiency of up to 53% in samples where a higher concentration of complexes was used.

### Example 5: Storing the surfaces of the invention

### Objective

To evaluate the possibility of storing the surfaces of the present invention.

### Method

The surfaces used in this study were Petri dishes (PD) 30 mm in diameter treated or not treated with PFM.
1. The protocol followed in the preceding examples was followed to cover the surfaces with PFM.
2. Solutions of lentiviral particles were prepared using 1.5 ml of frozen lentiviral supernatant (10⁸ TU/ml), adding it directly on the dishes. A lentiviral vector encoding GFP was used as the reporter gene.
3. The dishes were centrifuged for 1 hour at 3,000 g and 37°C.
4. The dishes were washed (5x50 ml) with a culture medium.
5. Part of the dishes was used directly in gene transfer experiments as detailed in Example 1. 0.1 ml of culture medium was added to the remaining dishes (per dish) and they were introduced in a -80°C freezer.
6. After the desired time (30 days), the dishes were taken out of the freezer and left at room temperature for 20 minutes to thaw. They were then washed with culture medium and cells were seeded thereon (HEK-293T, 5x10⁵ per well).
7. Forty eight hours after seeding, the cells were collected and analyzed by means of flow cytometry for quantifying GFP expression.

### Results

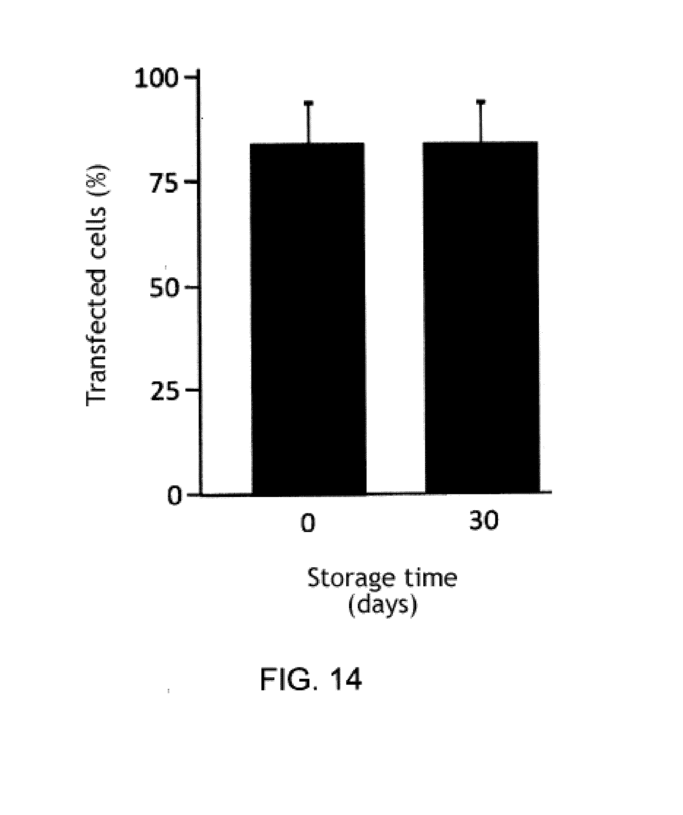

Figure 14 shows the efficiency of genetic transduction in stored dishes. It can be seen how after 30 days of storing at - 80°C, the dishes do not lose efficiency in a significant manner with respect to the first day. This confers the technology with great flexibility and ease of use.

### Example 6: Producing pluripotent cells by means of cellular reprogramming using the surfaces of the invention

### Objective

To produce pluripotent cells (induced Pluripotent Stem, iPS) from mouse embryonic fibroblasts (MEF) by means of cellular reprogramming using the surfaces of the invention.

### Method:

The dishes (PD, 30 mm) were modified with PFM according to the protocol described in preceding examples. The method of cellular reprogramming was the following:
1. Several culture dishes (30 mm) were seeded with MEF (1.5x10⁵ cells/dish) the day before starting the experiment. These cells will be used as positive control (see step 7).
2. 1.5 ml/dish of a lentiviral supernatant encoding the genes necessary for cellular reprogramming (in this case: Oct-4, Sox2, Klf4 and c-Myc) were added in the dishes treated with PFM.
3. The dishes were centrifuged for 1 hour at 3000 g and 37°C.
4. The dishes were washed with a culture medium (5x50 ml).
5. The dishes were incubated with 1 ml of 0.1% gelatin for 20 minutes at 37°C.
6. The dishes were again washed with a culture medium (5x50 ml).
7. The cells (MEF) were seeded at a density of 1.5x10⁵ cells/dish, and the lentiviral supernatant (1.5 ml/dish) was added to the positive control cells (see step 1). In these positive control cells, the supernatant was replaced with culture medium after 5 hours.
8. After four days, the cells were trypsinized and seeded on mitomycin-inactivated MEFs which were previously seeded in dishes of 100 mm.

The culture medium used was DMEM having a high glucose concentration (4.5 g/l) with glutamax and sodium pyruvate (GIBCO), 15% FBS, non-essential amino acids (1 mM), 0.1 mM betamercaptoethanol, 100 U/ml penicillin, 100 mg/ml streptomycin, 1,000 U/ml LIF (Leukemia Inhibitory Factor) and 2 mM valproic acid (valproic acid was only used on the first 7 days of culture).

### Results:

As can be seen in Figure 15, by using the technology of the present invention iPS cells were obtained with a greater efficiency with respect to those obtained with the original method of Takahashi and Yamanaka (Cell. 2006 126:663-676), in which lentiviral particles in suspension are added to the cells. Ten days after the initial hit, the number of colonies obtained by means of the CRS system was significantly (p<0.05) higher than in the control. Specifically, the number of transfected cells was 8.5 times higher. This corresponds to an efficiency of about one iPS colony for every 10,000 cells initially subjected to the hit. Figure 16 shows the results of the phenotypic characterization of the obtained colonies which confirm that the colonies are iPS cell colonies.

## Claims

1. A bioactive surface comprising:
a) a polymer matrix,
b) a connecting complex comprising at least one compound covalently bound to the surface of the polymer matrix (a), and
c) a gene transfer vector bound to the connecting complex (b),
where the compound of complex (b) is covalently bound to the transfer vector by means of a group selected from a carboxyl group, an amino group, an isocyanate group and a hydroxyl group.

2. The bioactive surface according to claim 1, where the compound bound to the matrix is bound to the transfer vector by means of a carboxyl group.

3. The bioactive surface according to claim 1, where the compound bound to the matrix is bound to the transfer vector by means of an amino group.

4. The bioactive surface according to any of claims 1 to 3, where the compound bound to the matrix has the following formula (I): 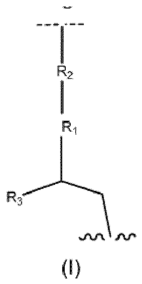
where: R₁ is a radical which may or may not exist and if it exists, it is selected from a group comprising a (C₁-C₆) alkyl, a (C₁-C₆) alkenyl, a cycloalkyl or an aryl;
R₂ is a carboxyl group (-COO-), an amino group (-NH-), an ether group (-O-) or an isocyanate group (-CON-), or
R₃ is a hydrogen, a (C₁-C₆) alkyl or a (C₁-C₆) alkene.

5. The bioactive surface according to claim 4, where the compound of formula (I) is:

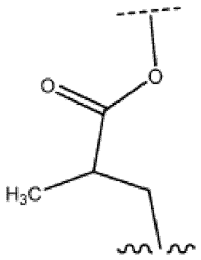

6. The bioactive surface according to any of claims 1 to 5, where the connecting complex further comprises a molecule selected from the list comprising: sugar, peptide, lipid or any combination thereof and where said molecule is located between the vector and the carboxyl group, amino group, isocyanate group and hydroxyl group of the compound covalently bound to the polymer matrix.

7. The bioactive surface according to any of claims 1 to 6, where the polymer matrix is selected from the list comprising: polystyrene, polycarbonate, poly(ethylene carbonate), polyethylene, polyolefins, poly(diol citrate), polyfumarate, polylactides, polycaprolactone, polyacrylamides, polysiloxanes, polyesters, polyamides, glycolic/lactic acid copolymers, polyurethane or any combination thereof.

8. The bioactive surface according to claim 7, where the polymer matrix is polystyrene.

9. The bioactive surface according to any of claims 1 to 8, where the gene transfer vector comprises at least one amino group or carboxyl group.

10. The bioactive surface according to claim 9, where the gene transfer vector comprises at least one amino group.

11. The bioactive surface according to any of claims 1 to 10, where the gene transfer vector is selected from the list comprising: nucleic acid-polyamine complex, nucleic acid-peptide complex, nucleic acid-polypeptide complex, nucleic acid-protein complex, nucleic acid-dendrimer complex, nucleic acid-lipid-polyamine complex, nucleic acid-modified inorganic nanoparticle complex, adenoviral vector, adeno-associated vector, retroviral vector, lentiviral vector, alpha viral vector, herpes viral vector, poxvirus-derived vector or coronavirus-derived vector.

12. The bioactive surface according to claim 11, where the gene transfer vector is a lentiviral vector.

13. The bioactive surface according to claim 11, where the gene transfer vector is an adenoviral vector.

14. An implantable device **characterized in that** at least one part of its surface is coated with the bioactive surface according to any of claims 1 to 13.

15. The implantable device according to claim 14, where said device is selected from the list comprising: a stent, an orthopedic prosthesis, a biocompatible membrane, a porous polymer, a patch, a suture, a capsule and a particle.

16. Use of the bioactive surface according to any of claims 1 to 13 or of the implantable device according to any of claims 14 or 15 for transferring a nucleic acid to at least one cell which contacts said bioactive surface.

17. Use according to claim 16, where the cell is a eukaryotic cell.

18. A method for transferring a nucleic acid to a cell which comprises contacting the bioactive surface according to any of claims 1 to 13 or the implantable device according to any of claims 14 or 15 with said cell.

19. The method according to claim 18, where the cell is a eukaryotic cell.

20. A kit for carrying out the method according to any of claim 18 or 19 comprising:
a) a polymer matrix,
b) a connecting complex comprising at least one compound covalently bound to the surface of the polymer matrix (a), and
c) at least one element necessary for forming a gene transfer vector.

21. The kit according to claim 20, where the connecting complex comprises a compound which is selected from the list comprising:

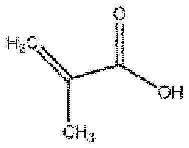

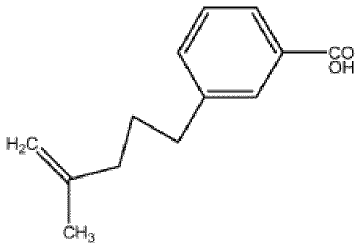

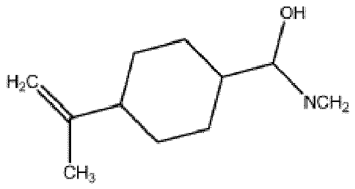

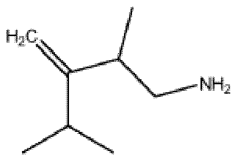

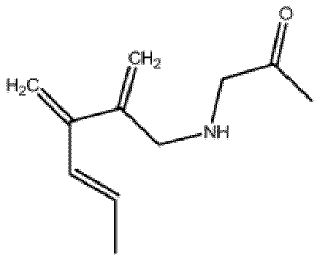

22. The kit for carrying out the method according to any of claims 16 or 17 comprising:
a) the bioactive surface according to any of claims 1 to 13 or the implantable device according to any of claims 14 or 15, and
b) at least one element necessary for forming a gene transfer vector.

23. The kit according to any of claims 19 to 21, where the element necessary for forming the gene transfer vector is selected from the list comprising: polyamine, peptide, polypeptide, protein, dendrimer, lipid-polyamine, modified inorganic nanoparticle and plasmid encoding an envelope protein of a viral vector.

24. The method for preparing the bioactive surface according to any of claims 1 to 13 which comprises contacting:
a polymer matrix comprising a connecting complex that is covalently bound to its surface by means of a compound containing a group selected from a carboxyl group, an amino group, an isocyanate group and a hydroxyl group, and a gene transfer vector.

25. The method according to claim 24, where the polymer matrix contacts the gene transfer vector at a temperature between 20 and 40°C for a time period between 5 minutes and 24 hours.

26. The method according to any of claims 24 or 25, where the gene transfer vector on the polymer matrix is centrifuged.

27. The method according to claim 25, where the gene transfer vector on the polymer matrix is centrifuged at a speed between 100 and 20,000 Xg.

28. The method according to any of claims 24 to 27, where the polymer matrix is subjected to a negative pressure.

29. The method according to claim 24, where the polymer matrix is subjected to a negative pressure of between -10⁻⁴ and-10⁻¹ mBar.

30. The method according to any of claims 24 to 29, where the polymer matrix is obtained by means of a method selected from the list comprising: chemical deposition, polymerization, plasma polymerization and iCVD.

31. The method according to any of claims 24 to 28, where the polymer matrix is obtained by:
a) activating the polymer matrix by means of cold plasma, and
b) passing a stream of connecting complex comprising at least one compound covalently bound to the surface of the polymer matrix obtained from step (a).

32. The method according to any of the preceding claims, where the connecting complex comprises a compound which is selected from the list comprising:

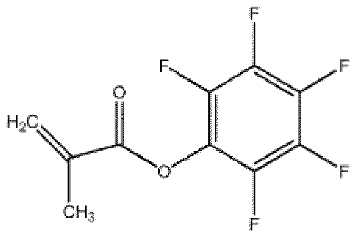

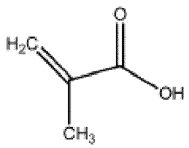

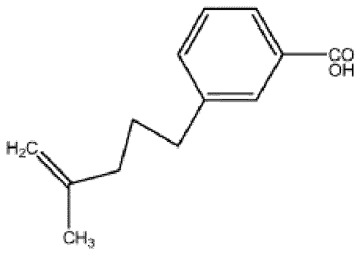

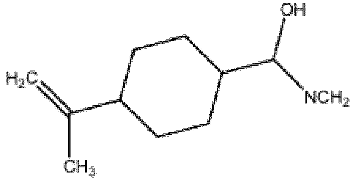

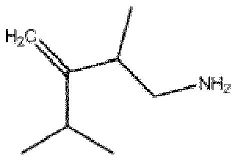

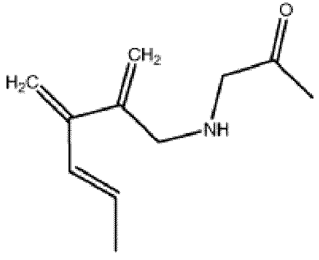

33. The method according to any of claims 31 or 32, where the connecting complex further comprises a molecule selected from the list comprising sugar, peptide, lipid or any combination thereof.
